# EUROPEAN PATENT SPECIFICATION

(11) **EP 3 916 084 B1**
(45) Date of publication and mention of the grant of the patent: **25.11.2026**
(21) Application number: 21166086.5
(22) Date of filing: 21.09.2015
(51) Int. Cl.: C12N 5/0783, C07K 14/715, C07K 14/54, C12N 15/867, A61K 35/17, A61P 35/00, A61K 40/11, A61K 40/31, A61K 40/42

(54) **CENTRAL MEMORY T CELLS FOR ADOPTIVE T CELL THERAPY**
ZENTRALE GEDÄCHTNIS-T-ZELLEN FÜR DIE ADOPTIVE T-ZELLTHERAPIE
LYMPHOCYTES T DE MÉMOIRE CENTRALE POUR THÉRAPIE ADOPTIVE PAR LYMPHOCYTES T

(30) Priority: 19.09.2014 US 201461053068 P
(43) Date of publication of application: 01.12.2021
(62) Document number(s) of the earlier application(s) in accordance with Art. 76 EPC: 15842405.1 / 3 200 591
(73) Proprietor: City of Hope, Duarte, CA 91010 (US)
(72) Inventor: BROWN, Christine E., Duarte, California 91010 (US); FORMAN, Stephen J., Duarte, California 91010 (US)
(74) Representative: Fish & Richardson P.C.

(56) References cited:
- EP-A1- 2 532 740
- WO-A1-2007/071053
- WO-A1-2012/024203
- WO-A1-2012/129514
- S. KONG ET AL: "Suppression of Human Glioma Xenografts with Second-Generation IL13R-Specific Chimeric Antigen Receptor-Modified T Cells", CLINICAL CANCER RESEARCH, vol. 18, no. 21, 1 November 2012 (2012-11-01), US, pages 5949 - 5960, XP055242650, ISSN: 1078-0432, DOI: 10.1158/1078-0432.CCR-12-0319
- KALOS MICHAEL ET AL: "T Cells with Chimeric Antigen Receptors Have Potent Antitumor Effects and Can Establish Memory in Patients with Advanced Leukemia", 20110810, vol. 3, no. 95, 10 August 2011 (2011-08-10), pages 95ra73.1, XP002667262, DOI: 10.1126/SCITRANSLMED.3002842

## Description

### BACKGROUND

Tumor-specific T cell based immunotherapies, including therapies employing engineered T cells, have been investigated for anti-tumor treatment. In some cases the T cells used in such therapies do not remain active *in vivo* for a long enough period. In some cases, the tumor-specificity of the T cells is relatively low. Therefore, there is a need in the art for tumor-specific cancer therapies with longer term anti-tumor functioning.

Adoptive T cell therapy (ACT) utilizing chimeric antigen receptor (CAR) engineered T cells may provide a safe and effective way to reduce recurrence rates of MG, since CAR T cells can be engineered to specifically recognize antigenically-distinct tumor populations (Cartellieri et al. 2010 J Biomed Biotechnol 2010:956304; Ahmed et al. 2010 Clin Cancer Res 16:474; Sampson et al. 2014 Clin Cancer Res 20:972; Brown et al. 2013 Clin Cancer Res 2012 18:2199; Chow et al. 2013 Mol Ther 21:629), and T cells can migrate through the brain parenchyma to target and kill infiltrative malignant cells (Hong et al. 2010 Clin Cancer Res 16:4892; Brown et al. 2007 J Immunal 179:3332; Hong et al. 2010 Clin Cancer Res 16:4892; Yaghoubi 2009 Nat Clin PRact Oncol 6:53). Preclinical studies have demonstrated that IL13Rα2-targeting CAR+ T cells exhibit potent major histocompatibility complex (MHC)-independent, IL13Rα2-specific cytolytic activity against both stem-like and differentiated glioma cells, and induce regression of established glioma xenografts *in vivo* (Kahlon et al. 2004 Cancer Res 64:9160; Brown et al. 2012 Clin Cancer Res 18:2199).
WO 2012/129514 A1 describes methods and compositions to confer or augment immune responses mediated by cellular immunotherapy, such as by adoptively transferring genetically modified tumor specific CD8+ T cells in the presence of tumor-specific, subset specific genetically modified CD4+ T cells, wherein the CD4+ T cells confer or augment a CD8+ T cell's ability to sustain anti-tumor reactivity and increase tumor-specific proliferation of the tumor-specific CD8+ T cells of interest.
WO 2007/071053 A1 describes methods, uses, products and kits relating to monitoring, assessing and modulating immune function and more particularly memory T cell function.

### SUMMARY

The present invention is defined by the independent claim. The dependent claims depict additional embodiments of the invention. Described herein are cell populations that comprise central memory T cells (Tcm). The cell populations include both CD4+ T cells and CD8+ T cells. The cells in the cell populations are useful for expressing chimeric transmembrane immunoreceptors (chimeric antigen receptors or "CARs") which comprise an extracellular domain (e.g., an scFv that binds to a selected target), a transmembrane region and an intracellular signaling domain, e.g., an intracellular domain that includes the signaling domain from the zeta chain of the human CD3 complex (CD3ζ) and one or more costimulatory domains, e.g., a 4-1BB costimulatory domain. The extracellular domain enables the CAR, when expressed on the surface of a T cell, to direct T cell activity to those cells expressing a target recognized by the extracellular domain. The inclusion of a costimulatory domain, such as the 4-1BB (CD137) costimulatory domain in series with CD3ζ in the intracellular region enables the T cell to receive co-stimulatory signals.

The Tcm cells described herein, for example, patient-specific, autologous or allogenic Tcm cells can be engineered to express a desired CAR and the engineered cells can be expanded and used in ACT. The population of T_{CM} cells are CD45RO+CD62L and include CD4+ and CD8+ cells.

The population of human T cells comprise a vector expressing a chimeric antigen receptor; the population of human central memory T cells (Tcm cells ) comprise at least 10% CD4+ cells and at least 10% CD8+ cells (e.g., at least 50% 60%, 70%, 80% of the cells are Tcm cells; at least 15%, 20%, 25%, 30%, 35% of the Tcm cells are CD4+ and at least 15%, 20%, 25%, 30%, 35% of the Tcm cells are CD8+ cells).

Described herein a population of human T cells transduced with a vector expressing a chimeric antigen receptor wherein at least 50% of the transduced human T cells are central memory T cells, wherein the central memory T cells are CD45RO+ and CD62L+ and comprise CD4+ and CD8+ cells, wherein at least 10% of the transduced cells central memory T cells are CD4+, wherein at least 10% of the transduced central memory T cells are CD8+, wherein the population of human T cells had been depleted of T cells that express CD25 and CD14 and cells that are CD45Ra+, wherein the depleted population of human T cells had been enriched for T cells expressing CD62L. In various embodiments: at least15% of the central memory T cells are CD4+ and at least 15% are CD8+; and at least 50% of the transduced human T cells are CD4+/CD8+/CD62L+.

Also described herein is a population of human T cells transduced with a vector expressing a chimeric antigen receptor wherein: at least 50% of the transduced human T cells are CD45R0+, CD62L+ and CD45Ra-, at least 10% of the cells are CD4+ and at least 10% of the cells are CD8+. In various embodiments: at least 10% of the cells that are CD45R0+, CD62L+ and CD45Ra- are also CD4+ and at least 10% of the cells that are CD45R0+, CD62L+ and CD45Ra- are also CD8+; and at least 15% of the cells that are CD45R0+, CD62L+ and CD45Ra- are also CD4+ and at least 15% of the cells that are CD45R0+, CD62L+ and CD45Ra- are also CD4+.

Also described is a pharmaceutical composition comprising the central memory T cells described herein for use in treating cancer in a patient in need thereof. The T cells can be autologous to the patient or are allogenic to the patient.

Also described is a method for preparing a population central memory T cells comprising: depleting a population of T cells obtained from a human subject for cells that express CD25, cells that express CD14, and cells that express CD45+ to prepare a depleted T cell population; enriching the depleted T cell population for cells expressing CD62L, thereby preparing a population of central memory T cells, wherein the method does not comprising a step of depleting a cell population for cells expressing CD4 and does not comprising a step of depleting a cell population of cells expressing CD8. In various embodiments: at least 50% (e.g., at least 60%, 70%, 80%, 90% or 95%) of the cells in the population central memory T cells are CD45R0+, CD62L+ and CD45Ra-, at least 10% of the cells are CD4+ and at least 10% of the cells are CD4+; at least 50% (e.g., at least 60%, 70%, 80%, 90% or 95%) of the cells in the population central memory T cells are CD45R0+, CD62L+ and CD45Ra-, at least 15% of the cells are CD4+ and at least 15% of the cells are CD4+; at least 50% of the cells in the population central memory T cells are CD45R0+, CD62L+ and CD45Ra-, at least 20% (e.g., at least 30%, 40% or 50%) of the cells are CD4+ and at least 20% (e.g., at least 30%, 40% or 50%) of the cells are CD4+. The method can further comprise stimulating the population of central memory T cells (e.g., by contacting the population of central memory T cells with CD3 and/or CD28); can comprise transducing the cells with a vector expressing a recombinant protein to create a population of genetically modified central memory T cells; and can comprise expanding the population of generically modified central memory T cells (e.g., of expanding the population of generically modified central memory T cells by exposing the cells to one or both of IL-2 and IL-15.

### DESCRIPTION OF DRAWINGS

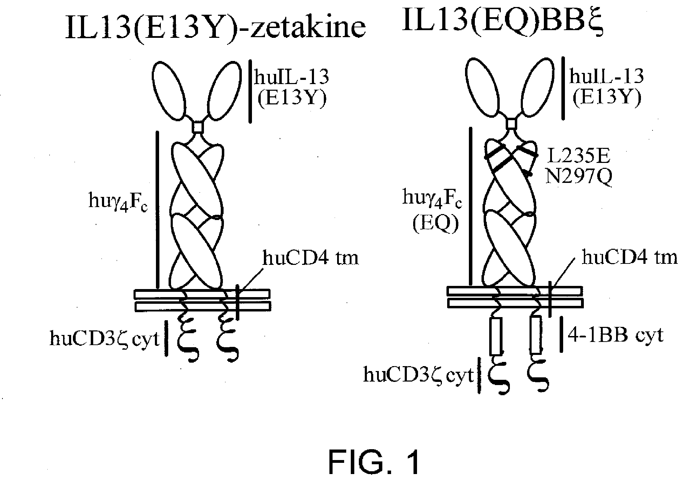

**Figure 1** is a schematic depiction of IL13(E13Y)-zetakine CAR (Left) composed of the IL13Rα2-specific human IL-13 variant (huIL-13(E13Y)), human IgG4 Fc spacer (huγ₄F_{c}), human CD4 transmembrane (huCD4 tm), and human CD3ζ chain cytoplasmic (huCD3ζ cyt) portions as indicated. Also depicted is a IL13(EQ)BBζ CAR which is the same as the IL13(E13Y)-zetakine with the exception of the two point mutations, L235E and N297Q indicated in red, that are located in the CH2 domain of the IgG4 spacer, and the addition of a costimulatory 4-1BB cytoplasmic domain (4-1BB cyt).

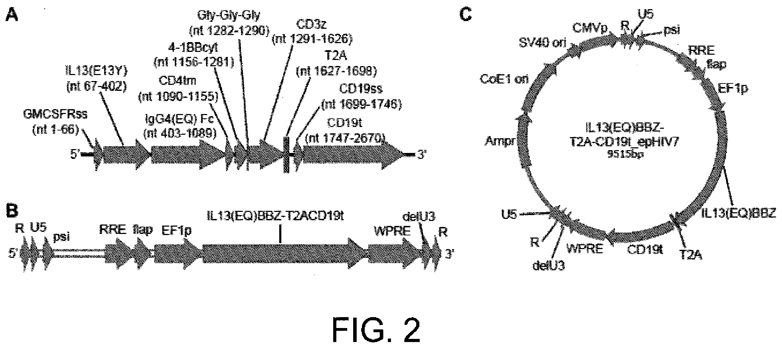

**Figures 2A-C** depict certain vectors an open reading frames. **A** is a diagram of the cDNA open reading frame of the 2670 nucleotide IL13(EQ)BBZ-T2ACD19t construct, where the IL13Rα2-specific ligand IL13(E13Y), IgG4(EQ) Fc hinge, CD4 transmembrane, 4-1BB cytoplasmic signaling, three-glycine linker, and CD3ζ cytoplasmic signaling domains of the IL13(EQ)BBZ CAR, as well as the T2A ribosome skip and truncated CD19 sequences are indicated. The human GM-CSF receptor alpha and CD19 signal sequences that drive surface expression of the IL13(EQ)BBζ CAR and CD19t are also indicated. **B** is a diagram of the sequences flanked by long terminal repeats (indicated by 'R') that will integrate into the host genome. **C** is a map of the IL 13 (EQ)BBZ-T2A-CD 19t_epHIV7 plasmid.

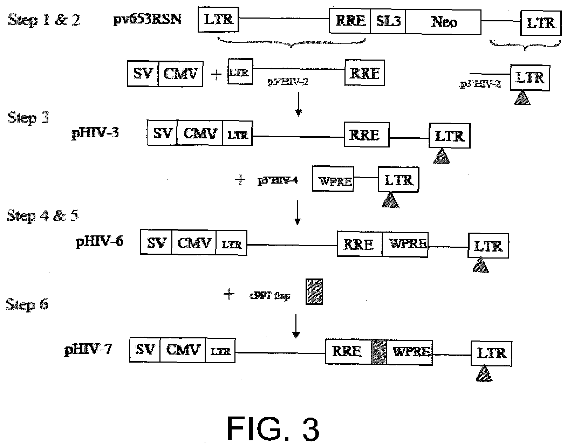

**Figure 3** depicts the construction of pHIV7.

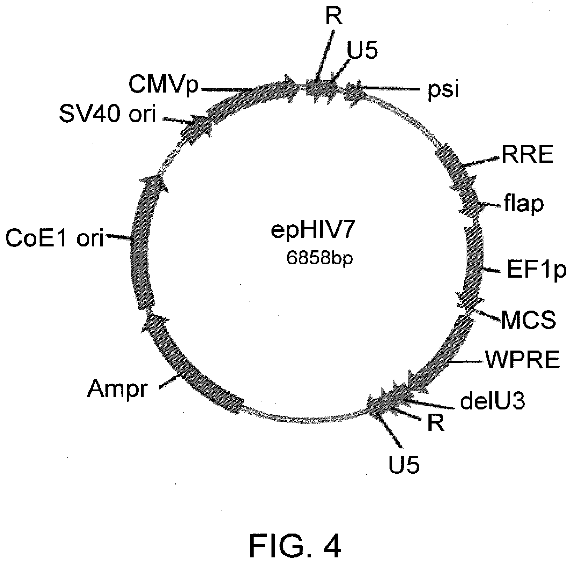

**Figure 4** depicts the elements of pHIV7.

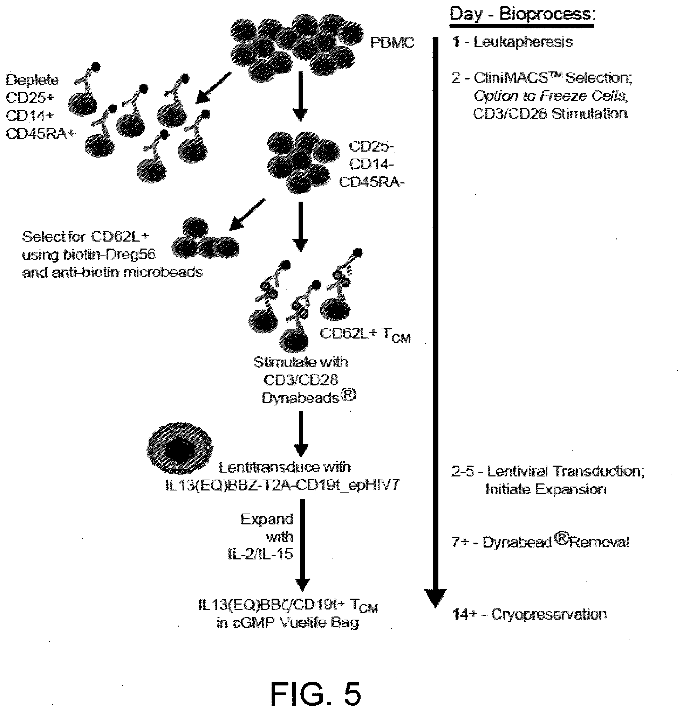

**Figure 5** depicts a production scheme for IL13(EQ)BBζ/CD19t+ T_{CM}.

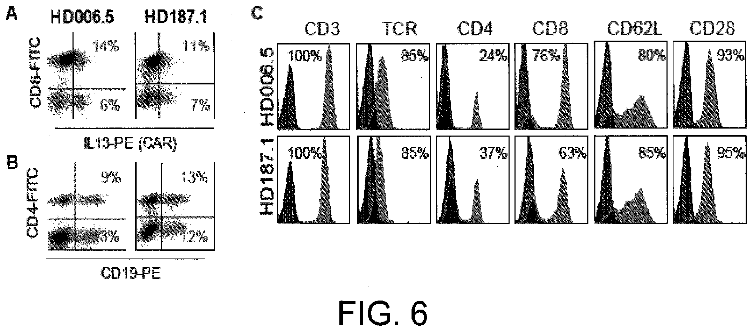

**Figures 6A-C** depicts the results of flow cytometric analysis of surface transgene and T cell marker expression. IL13(EQ)BBζ/CD19t+ T_{CM}HD006.5 and HD187.1 were co-stained with anti-IL13-PE and anti-CD8-FITC to detect CD8+ CAR+ and CD4+ (i.e., CD8 negative) CAR+ cells **(A),** or anti-CD19-PE and anti-CD4-FITC to detect CD4+ CD19t+ and CD8+ (i.e., CD4 negative) CAR+ cells **(B).** IL13(EQ)BBζ/CD19t+ T_{CM} HD006.5 and HD187.1 stained with fluorochromeconjugatedanti-CD3, TCR, CD4, CD8, CD62L and CD28 (grey histograms) or isotype controls (black histograms) **(C).** In all cases the percentages based on viable lymphocytes (DAPI negative) stained above isotype.

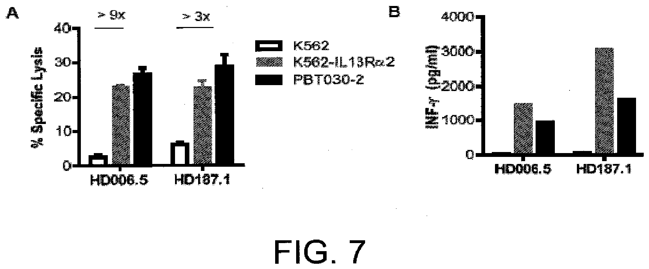

**Figures 7A-B** depict the *in vitro* functional characterization of IL13Rα2-specific effector function of IL13(EQ)BBZ+ T_{CM}. IL13(EQ)BBZ/CD19t+ T_{CM} HD006.5 and HD187.1 were used as effectors in a 6-hour ⁵¹Cr release assay using a 10:1 E:T ratio based on CD19t expression. The IL13Rα2-positive tumor targets were K562 engineered to express IL13Rα2 (K562-IL13Rα2) and primary glioma line PBT030-2, and the IL13Rα2-negative tumor target control was K562 parental line **(A).** IL13(EQ)BBZ/CD19t+ T_{CM} HD006. 5 and HD187.1 were evaluated for antigen-dependent cytokine production following overnight co-culture at a 10:1 E:T ratio with IL13Rα2-positive and negative targets. Cytokine levels were measured using the Bio-Plex Pro Human Cytokine TH1/TH2 Assay kit and INF-γ are reported **(B).**

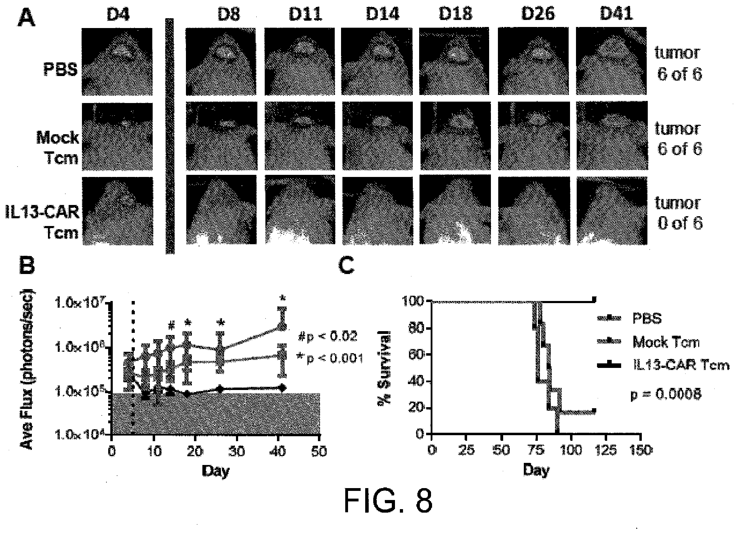

**Figures 8A-C** depict the result of studies demonstrating the regression of established glioma tumor xenografts after adoptive transfer of IL13(EQ)BBζ/CD19t+ T_{CM}. EGFP-ffLuc+ PBT030-2 tumor cells (1×10⁵) were stereotactically implanted into the right forebrain of NSG mice. On day 5, mice received either 2x10⁶ IL13(EQ)BBζ/CD19t+ T_{CM} (1.1x10⁶ CAR+; n=6), 2x10⁶ mock TCM (no CAR; n=6) or PBS (n=6). Representative mice from each group showing relative tumor burden using Xenogen Living Image **(A).** Quantification of ffLuc flux (photons/sec) shows that IL13(EQ)BBζ/CD19t+ T_{CM} induce tumor regression as compared to mock-transduced T_{CM} and PBS (#p<0.02, *p<0.001, repeated measures ANOVA) **(B).** Kaplan Meier survival curve (n=6 per group) demonstrating significantly improved survival (p=0.0008; log-rank test) for mice treated with IL13(EQ)BBζ/CD19t+ T_{CM} **(C).**

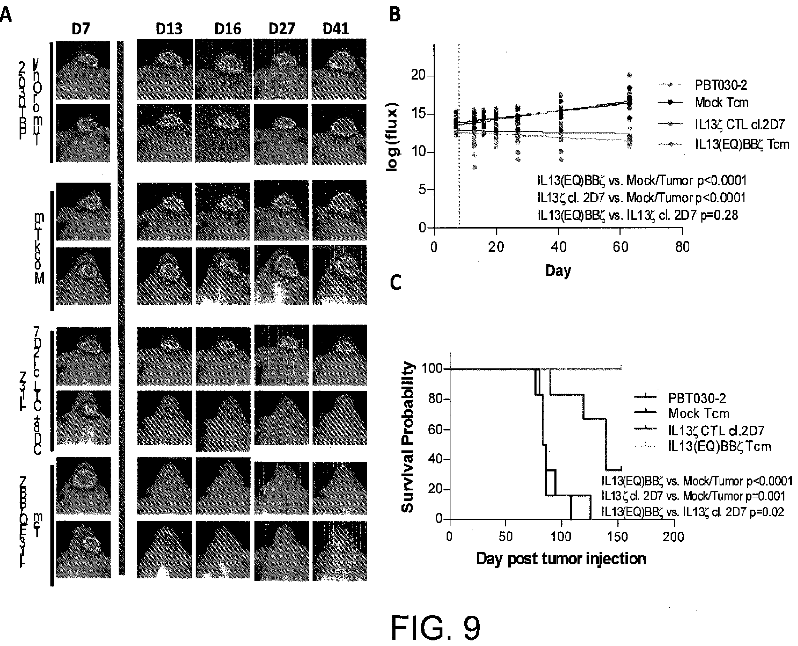

**Figures 9A-C** depict the results of studies comparing ant-tumor efficacy of IL13(EQ)BBZ T_{CM} and IL13-zetakine CTL clones. EGFP-ffLuc+ PBT030-2 TSs (1×10⁵) were stereotactically implanted into the right forebrain of NSG mice. On day 8, mice received either 1.6x10⁶ mock T_{CM} (no CAR), 1.0×10⁶ CAR+ IL13(EQ)BBζ T_{CM} (1.6×10⁶total T cells; 63% CAR), 1.0x10⁶ IL13-zetakine CD8+ CTL cl. 2D7 (clonal CAR+), or no treatment (n=6 per group). Representative mice from each group showing relative tumor burden using Xenogen Living Image **(A).** Linear regression lines of natural log of ffLuc flux (photons/sec) over time, P-values are for group by time interaction comparisons **(B).** Kaplan Meier survival analysis (n= 6 per group) demonstrate significantly improved survival (p=0.02; log-rank test) for mice treated with IL13(EQ)BBζ T_{CM} as compared to IL13-zetakine CD8+ CTL cl. **2D7 (C).**

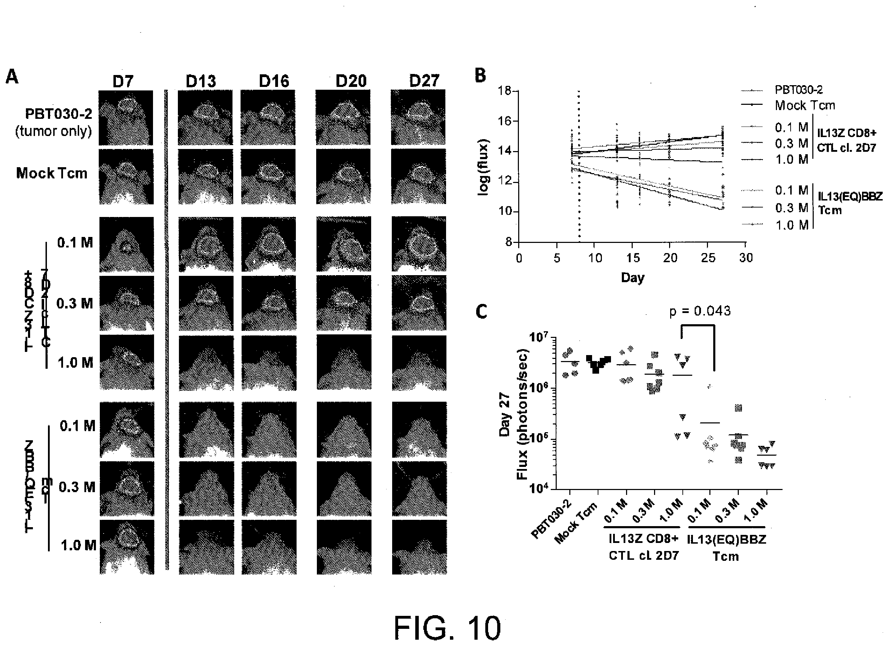

**Figures 10A-C** depict the results of studies comparing ant-tumor efficacy of IL13(EQ)BBζ T_{CM} and IL13-zetakine CTL clones. EGFP-ffLuc+ PBT030-2 TSs (1×10⁵) were stereotactically implanted into the right forebrain of NSG mice. On day 8, mice received either 1.3x10⁶ mock T_{CM} (no CAR; n=6), 1.0, 0.3 or 0.1x10⁶ CAR+ IL13(EQ)BBζ T_{CM} (78% CAR+; n=6-7), 1.0, 0.3 or 0.1x10⁶ IL13-zetakine CD8+ CTL cl. 2D7 (clonal CAR+; n=6-7), or no treatment (n=5). Xenogen imaging of representative mice from each group showing relative tumor burden **(A).** Linear regression lines of natural log of ffLuc flux (photons/sec) shows that IL13(EQ)BBζ T_{CM} achieve superior tumor regression as compared to first-generation IL13-zetakine CTL cl. 2D7, mock T_{CM} and tumor only **(B).** Average flux per group at day 27 post tumor injection demonstrating that the 0.1x10⁶ IL13(EQ)BBζ T_{CM} dose outperforms the ten-fold higher 1.0x10⁶ dose of IL13-zetakine CD8+ CTL cl. 2D7 (p = 0.043; Welch two sample t- test) **(C).**

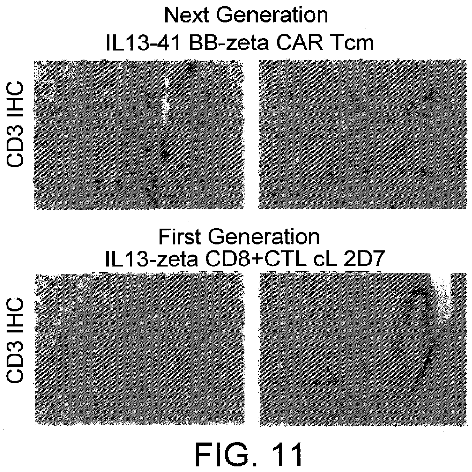

**Figure 11** depicts the results of studies demonstrating IL13(EQ)BBζ Tcm display improved persistence compared IL13-zetakine CTL clones. CD3 immunohistochemistry evaluating T cell persistence at the tumor site 7-days post T cell infusion. Significant numbers of T cells are detected for IL13(EQ)BBζ Tcm (top panel). By contrast, very few viable CD3+ IL13-zetakine T cells are detected (bottom panel).

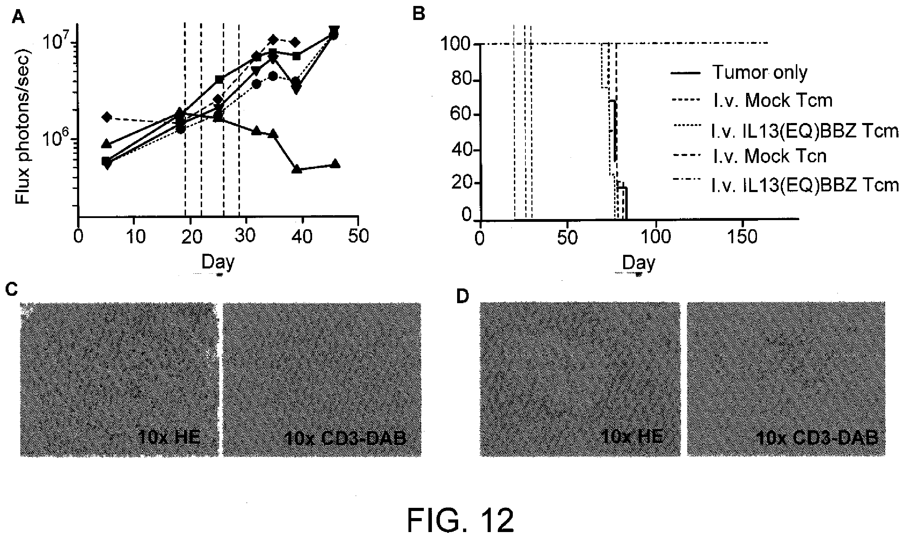

**Figures 12A-D** depict the results of experiments comparing route of CAR+ T cell delivery (i.c. versus i.v.) for large established tumors. EGFP-ffLuc+ PBT030-2 TSs (1×10⁵) were implanted into the right forebrain of NSG mice. On days 19 and 26, mice were injected i.v. through the tail vein with either 5x10⁶ CAR+ IL13(EQ)BBζ+ Tcm (11.8x10⁶ total cells; n=4), or mock Tcm (11.8x10⁶ cells; n=4). Alternatively, on days 19, 22, 26 and 29 mice were injected i.c. with either 1x10⁶ CAR+ IL13(EQ)BBζ+ Tcm (2.4x10⁶ total cells; n=4), or mock Tcm (2.4x10⁶ cells; n=5). Average ffLuc flux (photons/sec) over time shows that i.c. delivered IL13(EQ)BBζ Tcm mediates tumor regression of day 19 tumors. By comparison, i.v. delivered T cells do not shown reduction in tumor burden as compared to untreated or mock Tcm controls **(A).** Kaplan Meier survival curve demonstrates improved survival for mice treated i.c. IL13(EQ)BBZ Tcm as compared to mice treated with i.v. administered CAR+ Tcm (p = 0.0003 log rank test) **(B).** Representative H&E and CD3 IHC of mice treated i.v. **(C)** versus i.c. **(D)** with IL13(EQ)BBZ+ Tcm. CD3+ T cells were only detected in the i.c. treated group, with no CD3+ cells detected in the tumor or surrounding brain parenchyma for i.v. treated mice.

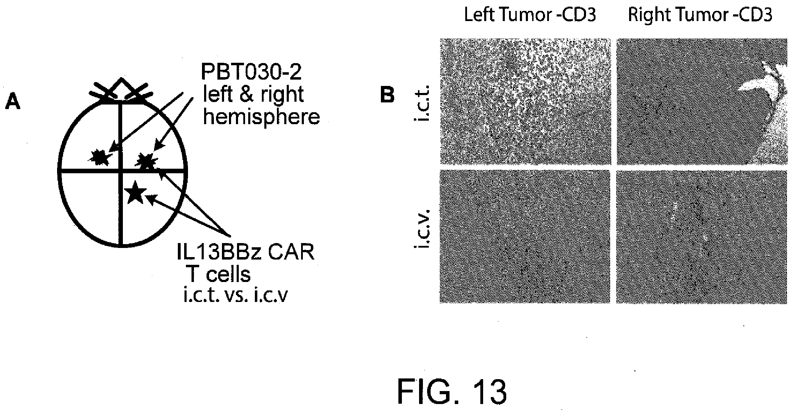

**Figures 13A-B** depict the results of studies showing that CAR+ T cell injected intracranially, either intratumoral (i.c.t.) or intraventricular (i.c.v.), can traffic to tumors on the opposite hemisphere. EGFP-ffLuc+ PBT030-2 TSs (1×10⁵) were stereotactically implanted into the right and left forebrains of NSG mice. On day 6, mice were injected i.c. at the right tumor site with 1.0x106 IL13(EQ)BBζ+ Tcm (1.6x106 total cells; 63% CAR; n=4). Schematic of multifocal glioma experimental model **(A).** CD3 IHC showing T cells infiltrating both the right and left tumor sites **(B).**

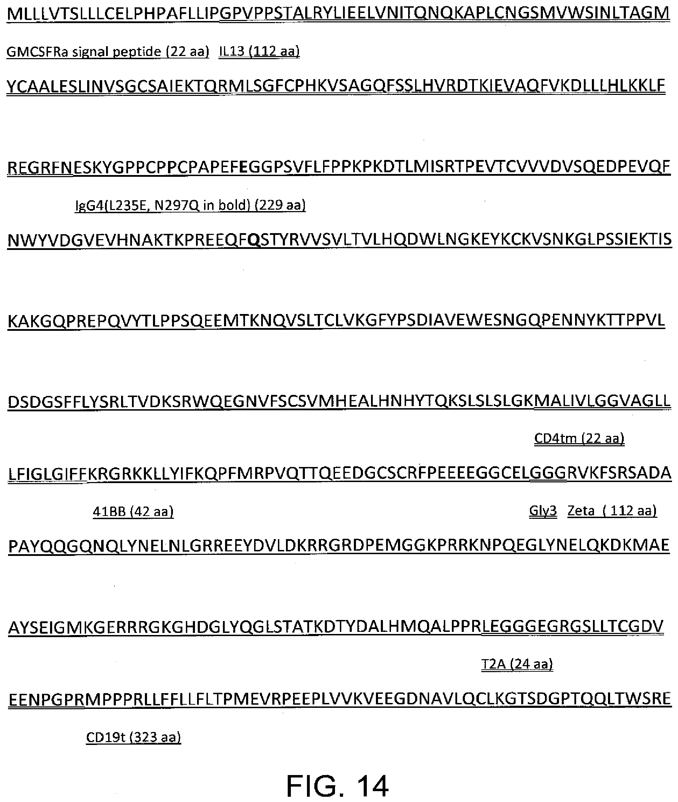

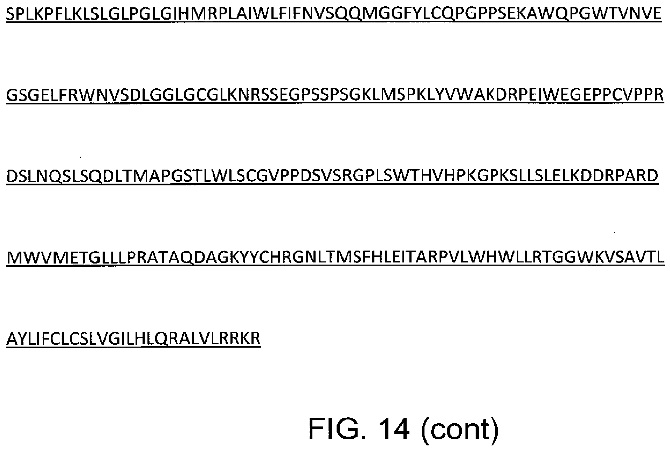

**Figure 14** depicts the amino acid sequence of IL13(EQ)BBζ/CD19t+ (SEQ ID NO:10).

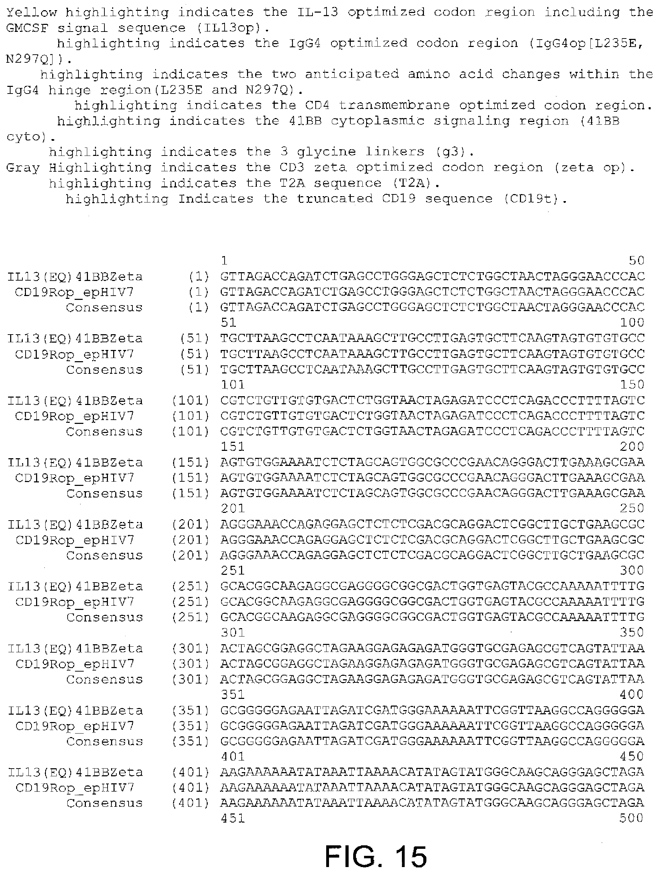

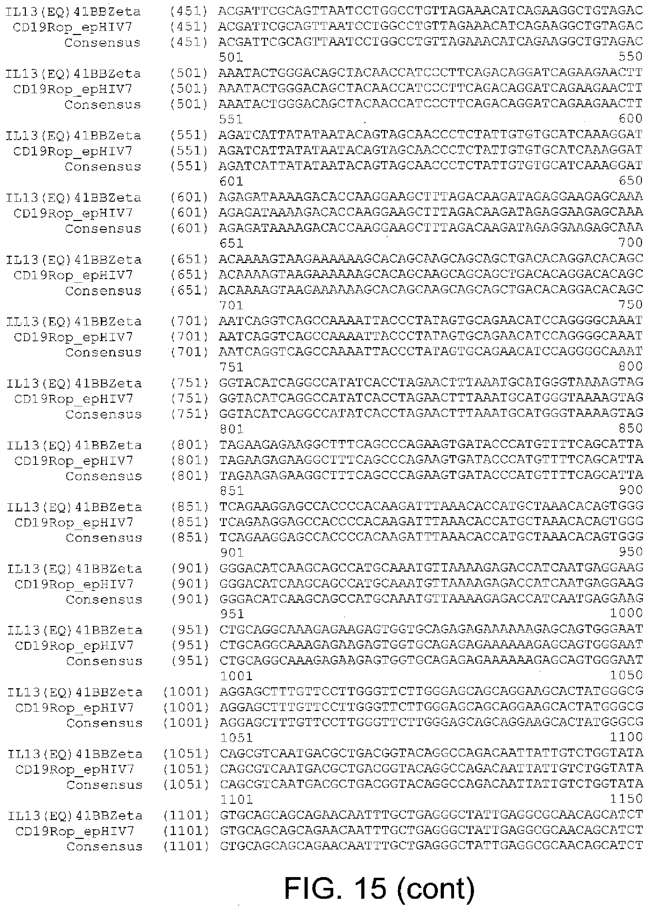

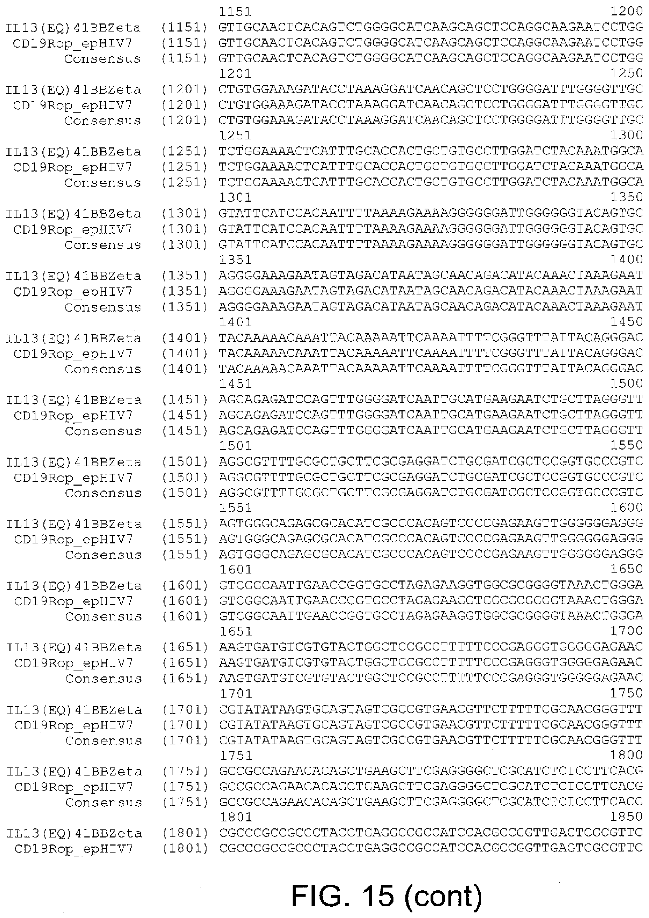

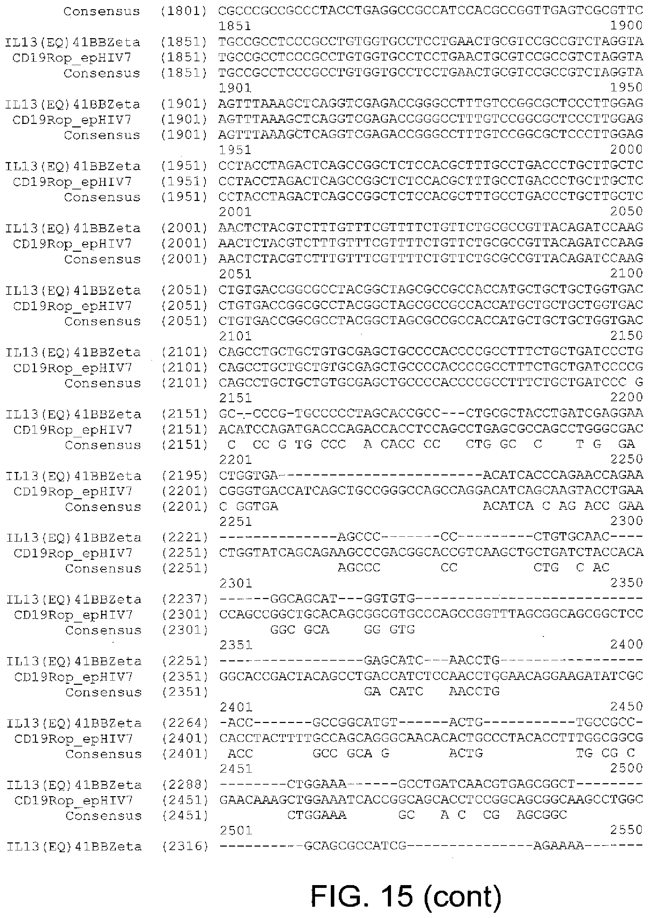

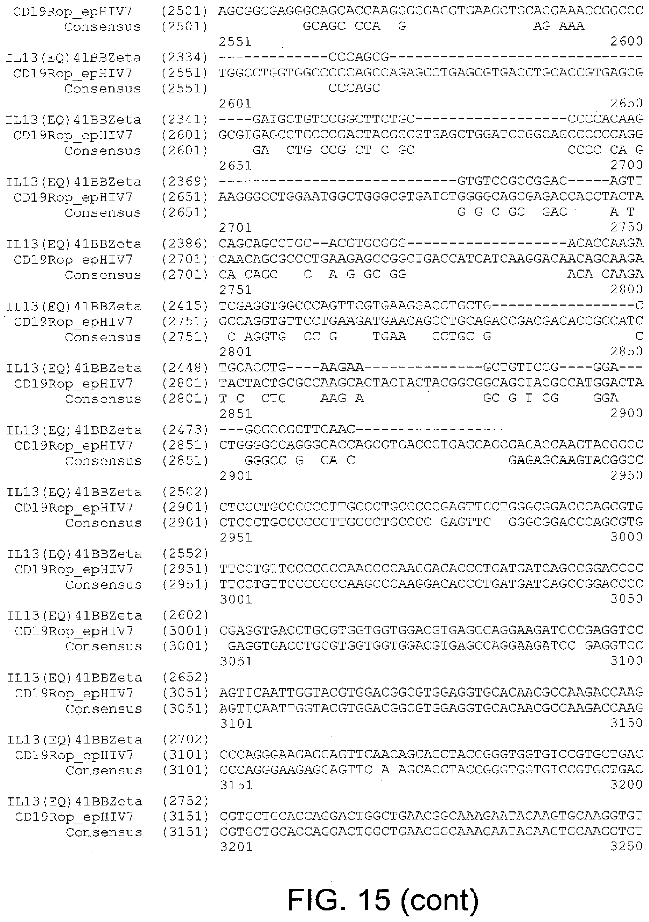

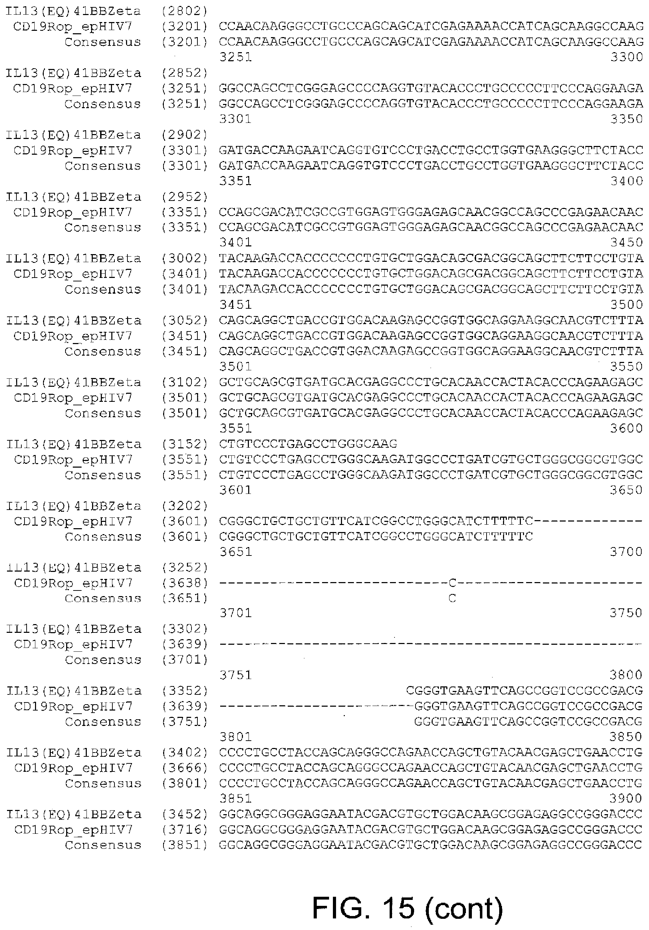

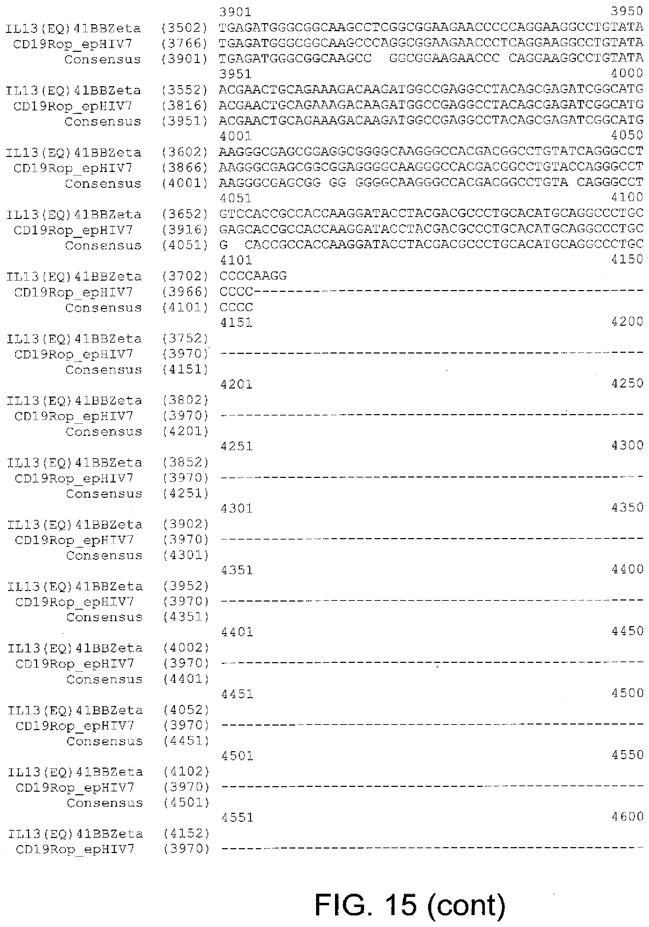

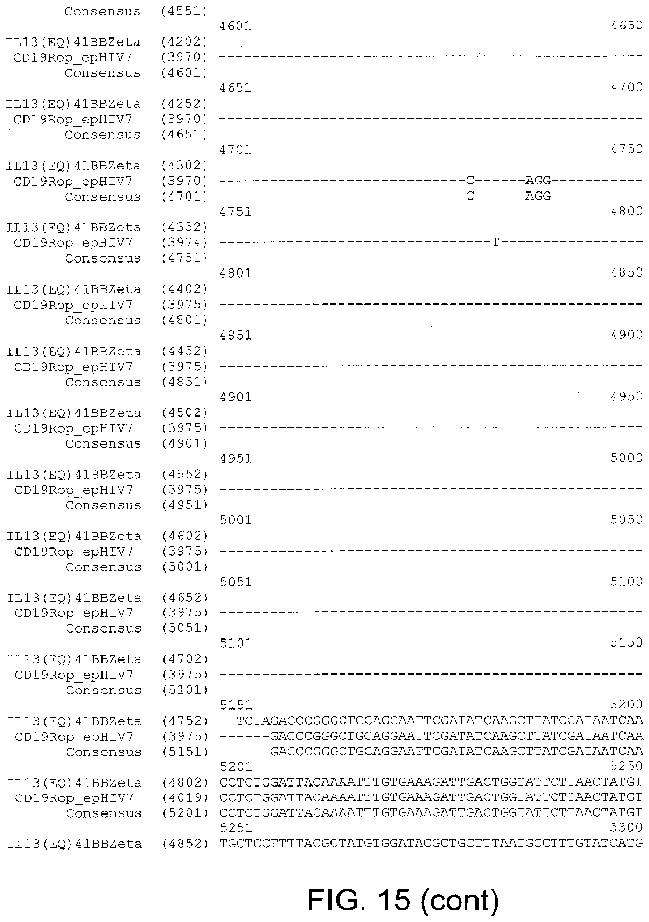

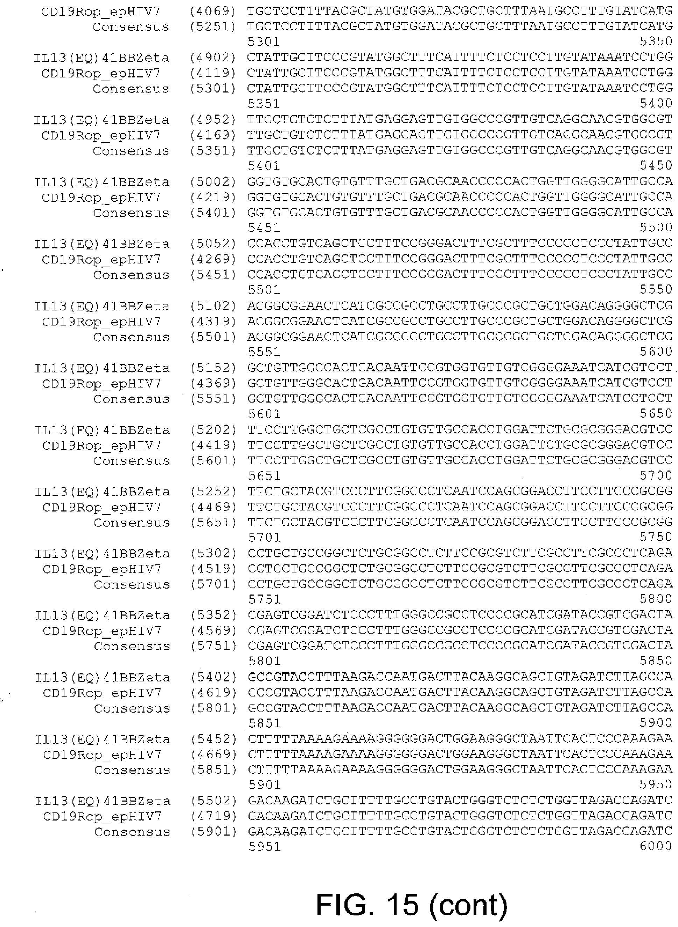

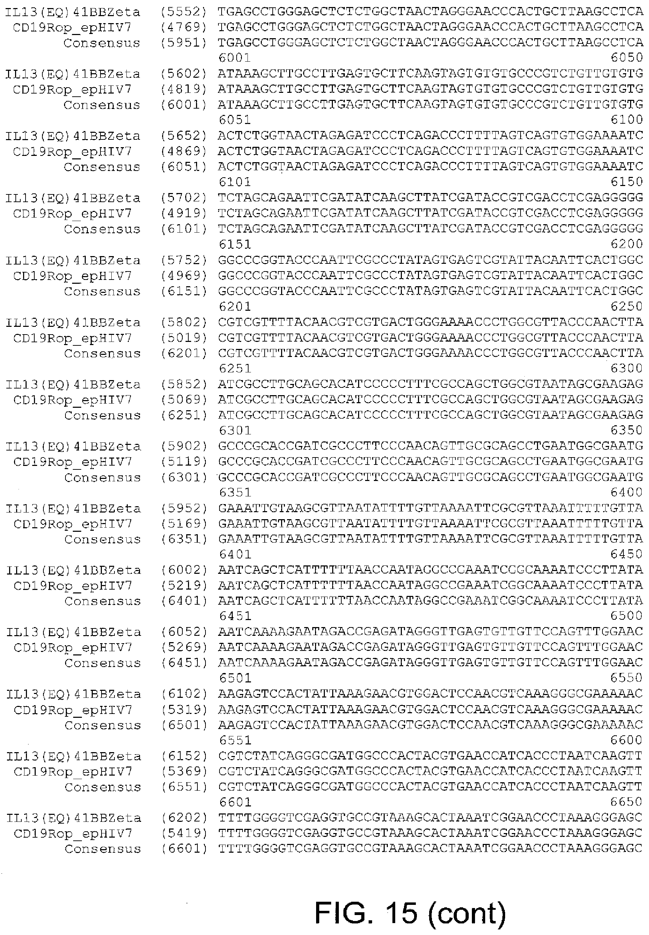

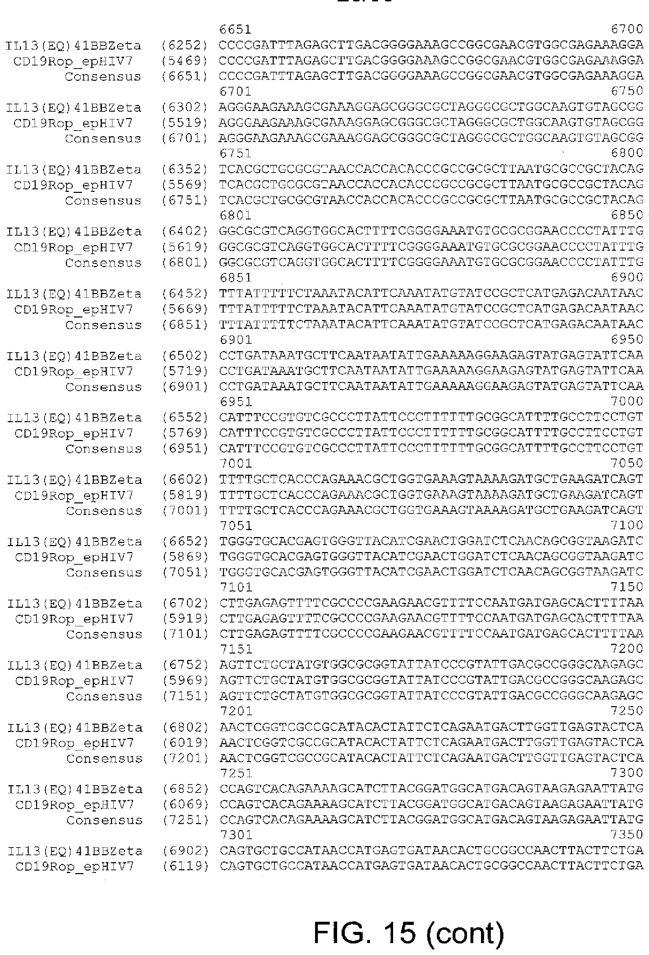

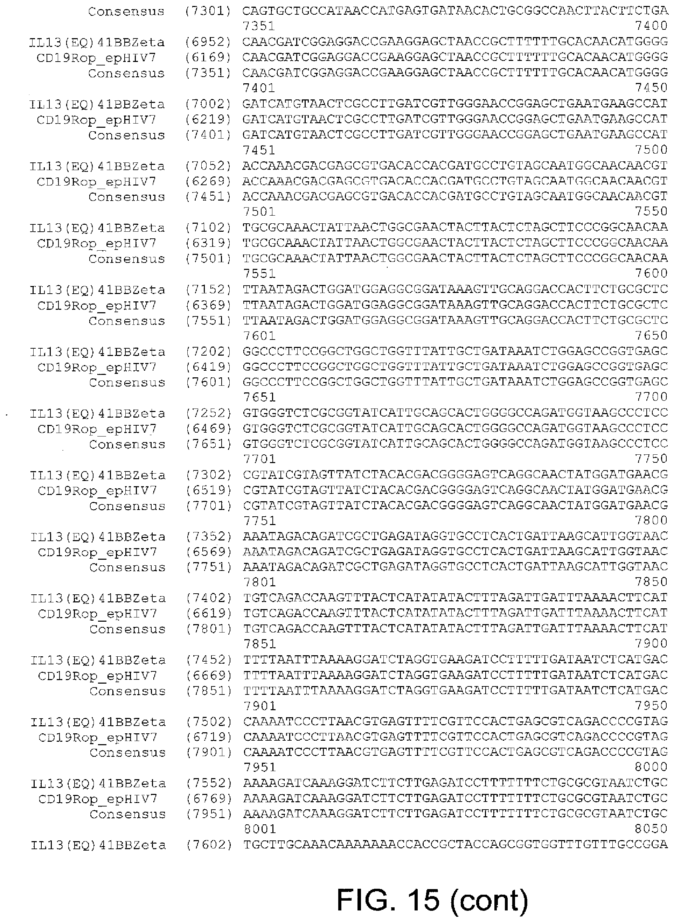

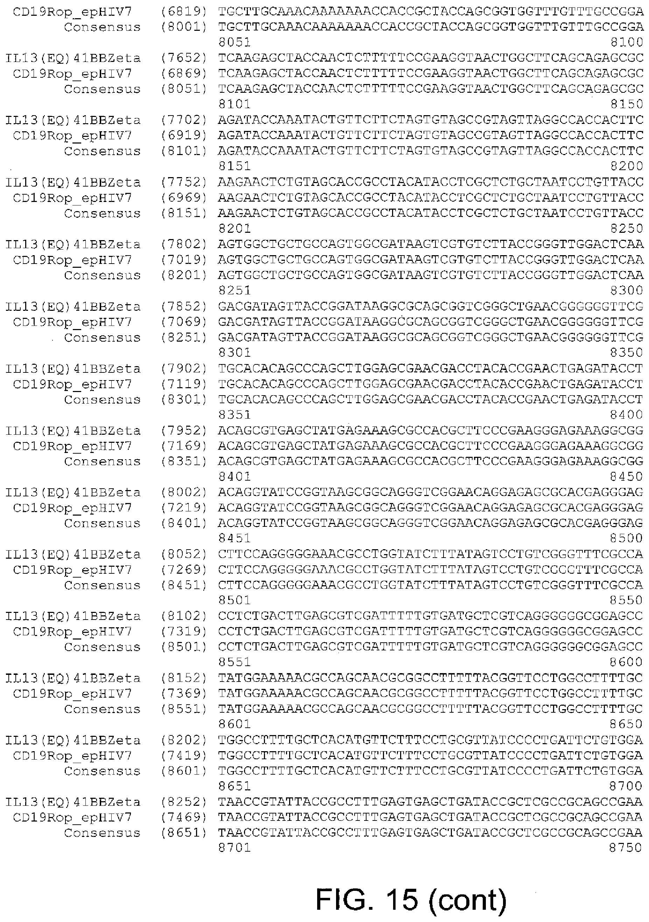

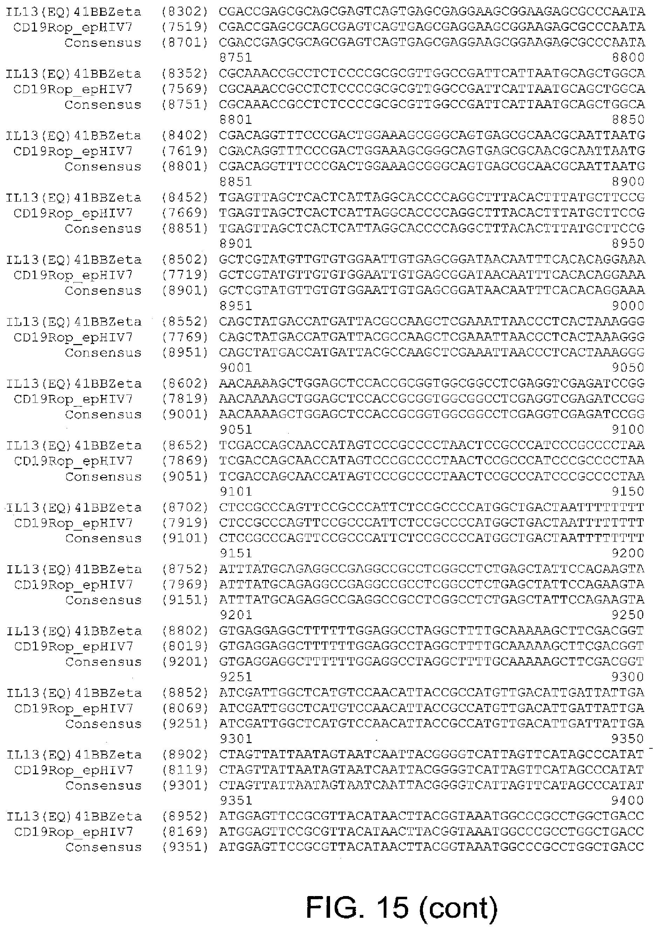

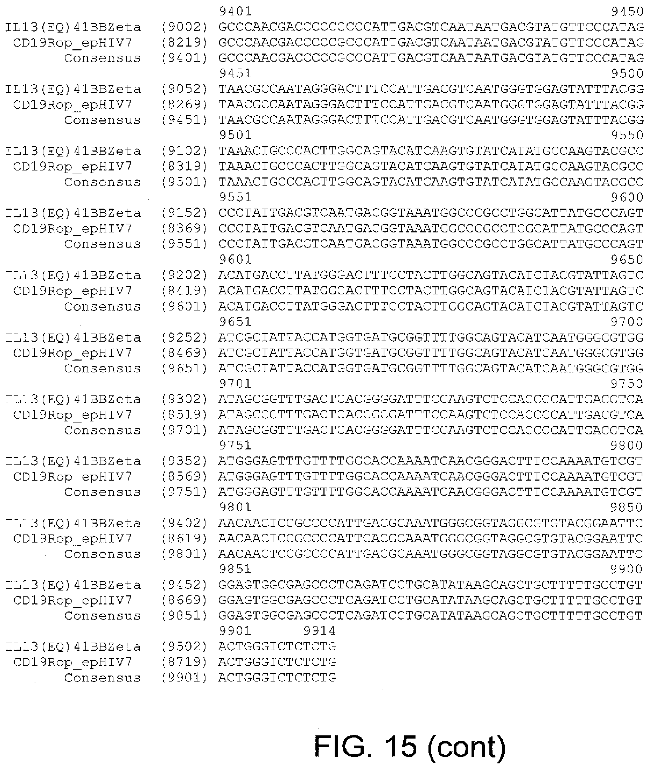

**Figure 15** depicts a sequence comparison of IL13(EQ)41BBζ[IL13 {EQ}41BBζ T2A-CD19t_epHIV7; pF02630] (SEQ ID NO: 12) and CD19Rop_epHIV7 (pJ01683) (SEQ ID NO: 13).

### DETAILED DESCRIPTION

A T_{CM} cell population that includes CD4+ cells and CD8+ cells ("T_{CM} CD4+/CD8+" cell population) is described below. The cells of the T_{CM} CD4+/CD8+ cell population can be activated with anti-CD3/CD28 and transduced with, for example, a SIN lentiviral vector that directs the expression of a CAR to create genetically modified cells. The activated/genetically modified cells can be expanded in vitro with IL-2/IL-15 and then used or cryopreserved and used later. Described is an example of the use of T_{CM} CD4+/CD8+ cell population to express a CAR targeted to IL13Rα2.

The CAR used in the examples below is referred to as IL13(EQ)BBζ. This CAR includes a variety of important features including: a IL13α2 ligand having an amino acid change that improves specificity of biding to IL13α2; the domain of CD137 (4-1BB) in series with CD3ζ to provide beneficial costimulation; and an IgG4 Fc region that is mutated at two sites within the CH2 region (L235E; N297Q) in a manner that reduces binding by Fc receptors (FcRs). This CAR and others can be produced using a vector in which the CAR open reading frame is followed by a T2A ribosome skip sequence and a truncated CD19 (CD19t), which lacks the cytoplasmic signaling tail (truncated at amino acid 323). In this arrangement, co-expression of CD19t provides an inert, non-immunogenic surface marker that allows for accurate measurement of gene modified cells, and enables positive selection of gene-modified cells, as well as efficient cell tracking and/or imaging of the therapeutic T cells in vivo following adoptive transfer. Co-expression of CD19t provides a marker for immunological targeting of the transduced cells in vivo using clinically available antibodies and/or immunotoxin reagents to selectively delete the therapeutic cells, and thereby functioning as a suicide switch.

Gliomas, express IL13 receptors, and in particular, high-affinity IL13 receptors. However, unlike the IL13 receptor, glioma cells overexpress a unique IL13Rα2 chain capable of binding IL13 independently of the requirement for IL4Rβ or γc44. Like its homolog IL4, IL13 has pleotropic immunoregulatory activity outside the CNS. Both IL13 and IL4 stimulate IgE production by B lymphocytes and suppress pro-inflammatory cytokine production by macrophages.

Detailed studies using autoradiography with radiolabeled IL13 have demonstrated abundant IL13 binding on nearly all malignant glioma tissues studied. This binding is highly homogeneous within tumor sections and in single cell analysis. However, molecular probe analysis specific for IL13Rα2 mRNA did not detect expression of the glioma-specific receptor by normal brain elements and autoradiography with radiolabeled IL13 also could not detect specific IL13 binding in the normal CNS. These studies suggest that the shared IL13Rα1/IL4β/γc receptor is not expressed detectably in the normal CNS. Therefore, IL13Rα2 is a very specific cell-surface target for glioma and is a suitable target for a CAR designed for treatment of a glioma.

Binding of IL13-based therapeutic molecules to the broadly expressed IL13Rα1/IL4β/γc receptor complex, however, has the potential of mediating undesired toxicities to normal tissues outside the CNS, and thus limits the systemic administration of these agents. An amino acid substitution in the IL13 alpha helix A at amino acid 13 of tyrosine for the native glutamic acid selectively reduces the affinity of IL13 to the IL13Rα1/IL4β/γc receptor. Binding of this mutant (termed IL13(E13Y)) to IL13Rα2, however, was increased relative to wild-type IL13. Thus, this minimally altered IL13 analog simultaneously increases IL13's specificity and affinity for glioma cells. Therefore, CAR described herein include an IL13 containing a mutation (E to Y or E to some other amino acid such as K or R or L or V) at amino acid 13 (according to the numbering of Debinski et al. 1999 Clin Cancer Res 5:3143s). IL13 having the natural sequence also may be used, however, and can be useful, particularly in situations where the modified T cells are to be locally administered, such as by injection directly into a tumor mass.

The CAR described herein can be produced by any means known in the art, though preferably it is produced using recombinant DNA techniques. Nucleic acids encoding the several regions of the chimeric receptor can be prepared and assembled into a complete coding sequence by standard techniques of molecular cloning known in the art (genomic library screening, PCR, primer-assisted ligation, site-directed mutagenesis, etc.) as is convenient. The resulting coding region is preferably inserted into an expression vector and used to transform a suitable expression host cell line, preferably a T lymphocyte cell line, and most preferably an autologous T lymphocyte cell line.

### Example 1: Construction and Structure of an IL13Rα2-specific CAR

The structure of a useful IL13Rα2-specific CAR is described below. The codon optimized CAR sequence contains a membrane-tethered IL-13 ligand mutated at a single site (E13Y) to reduce potential binding to IL13Rα1, an IgG4 Fc spacer containing two mutations (L235E; N297Q) that greatly reduce Fc receptor-mediated recognition models, a CD4 transmembrane domain, a costimulatory 4-1BB cytoplasmic signaling domain, and a CD3ζ cytoplasmic signaling domain. A T2A ribosome skip sequence separates this IL13(EQ)BBζ CAR sequence from CD19t, an inert, non-immunogenic cell surface detection/selection marker. This T2A linkage results in the coordinate expression of both IL13(EQ)BBζ and CD19t from a single transcript. **Figure 1A** is a schematic drawing of the 2670 nucleotide open reading frame encoding the IL13(EQ)BBZ-T2ACD19t construct. In this drawing, the IL13Rα2-specific ligand IL13(E13Y), IgG4(EQ) Fc, CD4 transmembrane, 4-1BB cytoplasmic signaling, three-glycine linker, and CD3ζ cytoplasmic signaling domains of the IL13(EQ)BBZ CAR, as well as the T2A ribosome skip and truncated CD19 sequences are all indicated. The human GM-CSF receptor alpha and CD19 signal sequences that drive surface expression of the IL13(EQ)BBZ CAR and CD19t are also indicated. Thus, the IL13(EQ)BBZ-T2ACD 19t construct includes a IL13Rα2-specific, hinge-optimized, costimulatory chimeric immunoreceptor sequence (designated IL13(EQ)BBZ), a ribosome-skip T2A sequence, and a CD19t sequence.

The IL13(EQ)BBZ sequence was generated by fusion of the human GM-CSF receptor alpha leader peptide with IL13(E13Y) ligand 5 L235E/N297Q-modified IgG4 Fc hinge (where the double mutation interferes with FcR recognition), CD4 transmembrane, 4-1BB cytoplasmic signaling domain, and CD3ζ cytoplasmic signaling domain sequences. This sequence was synthesized de novo after codon optimization. The T2A sequence was obtained from digestion of a T2A-containing plasmid. The CD19t sequence was obtained from that spanning the leader peptide sequence to the transmembrane components (i.e., basepairs 1-972) of a CD19-containing plasmid. All three fragments, 1) IL13(EQ)BBZ, 2) T2A, and 3) CD19t, were cloned into the multiple cloning site of the epHIV7 lentiviral vector. When transfected into appropriate cells, the vector integrates the sequence depicted schematically in **Figure 1B** into the host cells genome. **Figure 1C** provides a schematic drawing of the 9515 basepair IL13(EQ)BBZ-T2A-CD19t _epHIV7 plasmid itself.

As shown schematically in **Figure 2****,** IL13(EQ)BBZ CAR differs in several important respects from a previously described IL13Rα2-specific CAR referred to as IL13(E13Y)-zetakine (Brown et al. 2012 Clinical Cancer Research 18:2199). The IL13(E13Y)-zetakine is composed of the IL13Rα2-specific human IL-13 mutein (huIL-13(E13Y)), human IgG4 Fc spacer (huγ4Fc), human CD4 transmembrane (huCD4 tm), and human CD3ζ chain cytoplasmic (huCD3ζ cyt) portions as indicated. In contrast, the IL13(EQ)BBζ ) has two point mutations, L235E and N297Q that are located in the CH2 domain of the IgG4 spacer, and a costimulatory 4-1BB cytoplasmic domain (4-1BB cyt).

### Example 2: Construction and Structure of epHIV7 used for Expression of an IL13Rα2-specific CAR

The pHIV7 plasmid is the parent plasmid from which the clinical vector IL13(EQ)BBZ-T2A-CD19t_epHIV7 was derived in the T cell Therapeutics Research Laboratory (TCTRL) at City of Hope (COH). The epHIV7 vector used for expression of the CAR was produced from pHIV7 vector. Importantly, this vector uses the human EF1 promoter to drive expression of the CAR. Both the 5' and 3' sequences of the vector were derived from pv653RSN as previously derived from the HXBc2 provirus. The polypurine tract DNA flap sequences (cPPT) were derived from HIV-1 strain pNL4-3 from the NIH AIDS Reagent Repository. The woodchuck post-transcriptional regulatory element (WPRE) sequence was previously described.

Construction of pHIV7 is schematically depicted in **Figure 3****.** Briefly, pv653RSN, containing 653 bp from gag-pol plus 5' and 3' long-terminal repeats (LTRs) with an intervening SL3-neomycin phosphotransferase gene (Neo), was subcloned into pBluescript, as follows: In Step 1, the sequences from 5' LTR to rev-responsive element (RRE) made p5'HIV-1 51, and then the 5' LTR was modified by removing sequences upstream of the TATA box, and ligated first to a CMV enhancer and then to the SV40 origin of replication (p5'HIV-2). In Step 2, after cloning the 3' LTR into pBluescript to make p3'HIV-1, a 400-bp deletion in the 3' LTR enhancer/promoter was made to remove cis-regulatory elements in HIV U3 and form p3'HIV-2. In Step 3, fragments isolated from the p5'HIV-3 and p3'HIV-2 were ligated to make pHIV-3. In Step 4, the p3'HIV-2 was further modified by removing extra upstream HIV sequences to generate p3'HIV-3 and a 600-bp BamHI-SalI fragment containing WPRE was added to p3'HIV-3 to make the p3'HIV-4. In Step 5, the pHIV-3 RRE was reduced in size by PCR and ligated to a 5' fragment from pHIV-3 (not shown) and to the p3'HIV-4, to make pHIV-6. In Step 6, a 190-bp BglII-BamHI fragment containing the cPPT DNA flap sequence from HIV-1 pNL4-3 (55) was amplified from pNL4-3 and placed between the RRE and the WPRE sequences in pHIV6 to make pHIV-7. This parent plasmid pHIV7-GFP (GFP, green fluorescent protein) was used to package the parent vector using a four-plasmid system.

A packaging signal, psi ψ, is required for efficient packaging of viral genome into the vector. The RRE and WPRE enhance the RNA transcript transport and expression of the transgene. The flap sequence, in combination with WPRE, has been demonstrated to enhance the transduction efficiency of lentiviral vector in mammalian cells.

The helper functions, required for production of the viral vector), are divided into three separate plasmids to reduce the probability of generation of replication competent lentivirus via recombination: 1) pCgp encodes the gag/pol protein required for viral vector assembly; 2) pCMV-Rev2 encodes the Rev protein, which acts on the RRE sequence to assist in the transportation of the viral genome for efficient packaging; and 3) pCMV-G encodes the glycoprotein of the vesiculo-stomatitis virus (VSV), which is required for infectivity of the viral vector.

There is minimal DNA sequence homology between the pHIV7 encoded vector genome and the helper plasmids. The regions of homology include a packaging signal region of approximately 600 nucleotides, located in the gag/pol sequence of the pCgp helper plasmid; a CMV promoter sequence in all three helper plasmids; and a RRE sequence in the helper plasmid pCgp. It is highly improbable that replication competent recombinant virus could be generated due to the homology in these regions, as it would require multiple recombination events. Additionally, any resulting recombinants would be missing the functional LTR and tat sequences required for lentiviral replication.

The CMV promoter was replaced by the EF1α-HTLV promoter (EF1p), and the new plasmid was named epHIV7 **(****Figure 4****).** The EF1p has 563 bp and was introduced into epHIV7 using NruI and NheI, after the CMV promoter was excised.

The lentiviral genome, excluding gag/pol and rev that are necessary for the pathogenicity of the wild-type virus and are required for productive infection of target cells, has been removed from this system. In addition, the IL13(EQ)BBZ-T2ACD19t_epHIV7 vector construct does not contain an intact 3'LTR promoter, so the resulting expressed and reverse transcribed DNA proviral genome in targeted cells will have inactive LTRs. As a result of this design, no HIV-I derived sequences will be transcribed from the provirus and only the therapeutic sequences will be expressed from their respective promoters. The removal of the LTR promoter activity in the SIN vector is expected to significantly reduce the possibility of unintentional activation of host genes (56). Table 4 summarizes the various regulator elements present in IL13(EQ)BBZ-T2ACD 19t_epHIV7.

| **Table 4 Functional elements of IL13(EQ)41BBZ-T2A-CD19t_epHIV7** | | |
|---|---|---|
| **Regulatory Elements and Genes** | **Location (Nucleotide Numbers)** | **Comments** |
| U5 | 87-171 | 5' Unique sequence |
| psi | 233-345 | Packaging signal |
| RRE | 957-1289 | Rev-responsive element |
| flap | 1290-1466 | Contains polypurine track sequence and central termination sequence to facilitate nuclear import of pre-integration complex |
| EF1p Promoter | 1524-2067 | EF1-alpha Eukaryotic Promoter sequence driving expression of CD 19Rop |
| IL1-IgG4 (EQ)-41BB-Zeta-T2A- | 2084-4753 | Therapeutic insert |

| **Table 4 Functional elements of IL13(EQ)41BBZ-T2A-CD19t_epHIV7** | | |
|---|---|---|
| **Regulatory Elements and Genes** | **Location (Nucleotide Numbers)** | **Comments** |
| CD19t | | |
| WPRE | 4790-5390 | Woodchuck hepatitis virus derived regulatory element to enhance viral RNA transportation |
| delU3 | 5405-5509 | 3' U3 with deletion to generate SIN vector |
| R | 5510-5590 | Repeat sequence within LTR |
| U5 | 5591-5704 | 3' U5 sequence in LTR |
| Amp^{R} | 6540-7398 | Ampicillin-resistance gene |
| CoE1 ori | 7461-8342 | Replication origin of plasmid |
| SV40 ori | 8639-8838 | Replication origin of SV40 |
| CMV promoter | 8852-9451 | CMV promoter to generate viral genome RNA |
| R | 9507-86 | Repeat sequence within LTR |

### Example 3: Production of Vectors for Transduction of Patient T Cells

For each plasmid (IL13(EQ)BBZ-T2A-CD19t_epHIV7; pCgp; pCMV-G; and pCMV-Rev2), a seed bank is generated, which is used to inoculate the fermenter to produce sufficient quantities of plasmid DNA. The plasmid DNA is tested for identity, sterility and endotoxin prior to its use in producing lentiviral vector.

Briefly, cells were expanded from the 293T working cell (WCB), which has been tested to confirm sterility and the absence of viral contamination. A vial of 293T cells from the 293T WCB was thawed. Cells were grown and expanded until sufficient numbers of cells existed to plate an appropriate number of 10 layer cell factories (CFs) for vector production and cell train maintenance. A single train of cells can be used for production.

The lentiviral vector was produced in sub-batches of up to 10 CFs. Two sub-batches can be produced in the same week leading to the production of approximately 20 L of lentiviral supernatant/week. The material produced from all sub-batches were pooled during the downstream processing phase, in order to produce one lot of product. 293T cells were plated in CFs in 293T medium (DMEM with 10% FBS). Factories were placed in a 37°C incubator and horizontally leveled in order to get an even distribution of the cells on all the layers of the CF. Two days later, cells were transfected with the four lentiviral plasmids described above using the CaPO4 method, which involves a mixture of Tris:EDTA, 2M CaCl2, 2X HBS, and the four DNA plasmids. Day 3 after transfection, the supernatant containing secreted lentiviral vectors was collected, purified and concentrated. After the supernatant was removed from the CFs, End-of-Production Cells were collected from each CF. Cells were trypsinized from each factory and collected by centrifugation. Cells were resuspended in freezing medium and cryopreserved. These cells were later used for replication-competent lentivirus (RCL) testing.

To purify and formulate vectors crude supernatant was clarified by membrane filtration to remove the cell debris. The host cell DNA and residual plasmid DNA were degraded by endonuclease digestion (Benzonase^{®}). The viral supernatant was clarified of cellular debris using a 0.45 µm filter. The clarified supernatant was collected into a preweighed container into which the Benzonase^{®} is added (final concentration 50 U/mL). The endonuclease digestion for residual plasmid DNA and host genomic DNA as performed at 37°C for 6 h. The initial tangential flow ultrafiltration (TFF) concentration of the endonuclease-treated supernatant was used to remove residual low molecular weight components from the crude supernatant, while concentrating the virus ~20 fold. The clarified endonuclease-treated viral supernatant was circulated through a hollow fiber cartridge with a NMWCO of 500 kD at a flow rate designed to maintain the shear rate at ~4,000 sec-1 or less, while maximizing the flux rate. Diafiltration of the nuclease-treated supernatant was initiated during the concentration process to sustain the cartridge performance. An 80% permeate replacement rate was established, using 4% lactose in PBS as the diafiltration buffer. The viral supernatant was brought to the target volume, representing a 20-fold concentration of the crude supernatant, and the diafiltration was continued for 4 additional exchange volumes, with the permeate replacement rate at 100%.

Further concentration of the viral product was accomplished by using a high speed centrifugation technique. Each sub-batch of the lentivirus was pelleted using a Sorvall RC-26 plus centrifuge at 6000 RPM (6,088 RCF) at 6oC for 16-20 h. The viral pellet from each sub-batch was then reconstituted in a 50 mL volume with 4% lactose in PBS. The reconstituted pellet in this buffer represents the final formulation for the virus preparation. The entire vector concentration process resulted in a 200-fold volume reduction, approximately. Following the completion of all of the sub-batches, the material was then placed at -80oC, while samples from each sub-batch were tested for sterility. Following confirmation of sample sterility, the sub-batches were rapidly thawed at 37oC with frequent agitation. The material was then pooled and manually aliquoted in the Class II Type A/B3 biosafety cabinet in the viral vector suite. A fill configuration of 1 mL of the concentrated lentivirus in sterile USP class 6, externally threaded O-ring cryovials was used. Center for Applied Technology Development (CATD)'s Quality Systems (QS) at COH released all materials according to the Policies and Standard Operating Procedures for the CBG and in compliance with current Good Manufacturing Practices (cGMPs).

To ensure the purity of the lentiviral vector preparation, it was tested for residual host DNA contaminants, and the transfer of residual host and plasmid DNA. Among other tests, vector identity was evaluated by RT-PCR to ensure that the correct vector is present. All release criteria were met for the vector intended for use in this study.

### Example 4: Preparation of a T_{CM} CD4+/CD8+ Cell Population Suitable for Use in ACT

An outline of the manufacturing strategy for a T_{CM} CD4+/CD8+ cell population is depicted in **Figure 8** (Manufacturing schema for IL13(EQ)BBζ/CD19t+ T_{CM}). Specifically, apheresis products obtained from consented research participants are ficolled, washed and incubated overnight. Cells are then depleted of monocyte, regulatory T cell and naïve T cell populations using GMP grade anti-CD 14, anti-CD25 and anti-CD45RA reagents (Miltenyi Biotec) and the CliniMACS^{™} separation device. Following depletion, negative fraction cells are enriched for CD62L+ T_{CM} cells using DREG56-biotin (COH clinical grade) and anti-biotin microbeads (Miltenyi Biotec) on the CliniMACSTM separation device. The cells are not depleted for CD4+ cells or for CD8+ cells.

Following enrichment, T_{CM} CD4+/CD8+ cells are formulated in complete X-Vivo15 plus 50 IU/mL IL-2 and 0.5 ng/mL IL-15 and transferred to a Teflon cell culture bag, where they are stimulated with Dynal ClinEx^{™} Vivo CD3/CD28 beads. Up to five days after stimulation, cells are transduced with a desired vector expressing a CAR (e.g., IL13(EQ)BBZ-T2A-CD19t_epHIV7 lentiviral vector) at a multiplicity of infection (MOI) of, for example, 1.0 to 0.3. Cultures are maintained for up to 42 days with addition of complete X-Vivo15 and IL-2 and IL-15 cytokine as required for cell expansion (keeping cell density between 3x10⁵ and 2x10⁶ viable cells/mL, and cytokine supplementation every Monday, Wednesday and Friday of culture). Cells typically expand to approximately 10⁹ cells under these conditions within 21 days. At the end of the culture period cells are harvested, washed twice and formulated in clinical grade cryopreservation medium (Cryostore CS5, BioLife Solutions).

On the day(s) of T cell infusion, the cryopreserved and released product is thawed, washed and formulated for re-infusion. The cryopreserved vials containing the released cell product are removed from liquid nitrogen storage, thawed, cooled and washed with a PBS/2% human serum albumin (HSA) Wash Buffer. After centrifugation, the supernatant is removed and the cells resuspended in a Preservative-Free Normal Saline (PFNS)/ 2% HSA infusion diluent. Samples are removed for quality control testing.

Two qualification runs on cells procured from healthy donors were performed using the manufacturing platform described above. Each preclinical qualification run product was assigned a human donor (HD) number - HD006.5 and HD187.1. Importantly, as shown in Table 5, these qualification runs expanded >80 fold within 28 days and the expanded cells expressed the IL13(EQ)BBγ/CD19t transgenes.

**Table 5: Summary of Expression Data from Pre-clinical Qualification Run Product**

| **Cell Product** | **CAR** | **CD19** | **CD4+** | **CD8+** | **Fold Expansion** |
|---|---|---|---|---|---|
| HD006.5 | 20% | 22% | 24% | 76% | 84-fold (28 days) |
| Hd187.1 | 18% | 25% | 37% | 63% | 259-fold (28 days) |

### Example 5: Flow cytometric analysis of surface transgene and T cell marker expression in IL13(EQ)BBγ/CD19t+T_{CM}

The two preclinical qualification run products described in Example 4 were used in pre-clinical studies to as described below. **Figures 6A-C** depict the results of flow cytometric analysis of surface transgene and T cell marker expression. IL13(EQ)BBγ/CD19t+ T_{CM}HD006.5 and HD187.1 were co-stained with anti-IL13-PE and anti-CD8-FITC to detect CD8+ CAR+ and CD4+ (i.e., CD8 negative) CAR+ cells **(****Figure 6A****),** or anti-CD19-PE and anti-CD4-FITC to detect CD4+ CD19t+ and CD8+ (i.e., CD4 negative) CAR+ cells **(****Figure 6B****).** IL13(EQ)BBγ/CD19t+ T_{CM}HD006.5 and HD187.1 were stained with fluorochrome-conjugated anti-CD3, TCR, CD4, CD8, CD62L and CD28 (grey histograms) or isotype controls (black histograms). **(****Figure 6C****).** In each of **Figures 6A-C,** the percentages indicated are based on viable lymphocytes (DAPI negative) stained above isotype.

### Example 6: Effector Activity of IL13(EQ)BBγ/CD19t+ T_{CM}

The effector activity of IL13(EQ)BBζ/CD19t+ T_{CM} was assessed and the results of this analysis are depicted in **Figures 7A-B.** Briefly, IL13(EQ)BBγ/CD19t+ T_{CM} HD006.5 and HD187.1 were used as effectors in a 6-hour 51Cr-release assay using a 10E:1T ratio based on CD19t expression. The IL13Rα2-positive tumor targets were K562 engineered to express IL13Rα2 (K562-IL13Rα2) and primary glioma line PBT030-2, and the IL13Rα2-negative tumor target control was the K562 parental line **(****Figure 7A****).** IL13(EQ)BBγ/CD19t+ HD006.5 and HD187.1 were evaluated for antigen-dependent cytokine production following overnight co-culture at a 10E:1T ratio with the same IL13Rα2-positive and negative targets as described in above. Cytokine levels were measured using the Bio-Plex Pro Human Cytokine TH1/TH2 Assay kit and INF-γ levels are depicted **(****Figure 7B****).**

### Example 7: In vivo Anti-tumor Activity of IL 13 (EQ)BBγ/CD19t+ T_{CM}

The studies described below demonstrate that IL13(EQ)BBγ/CD19t+ T_{CM} exhibit anti-tumor efficacy in *in vivo* mouse models. Specifically, we have evaluated the anti-tumor potency of IL13(EQ)BBγ/CD19t+ T_{CM} against the IL13Rα2+ primary low-passage glioblastoma tumor sphere line PBT030-2, which has been engineered to express both EGFP and firefly luciferase (ffLuc) reporter genes (PBT030-2 EGFP:ffLuc) (**6**). A panel of primary lines (PBT) from patient glioblastoma specimens grown as tumor spheres (TSs) in serum-free media. These expanded TS lines exhibit stem cell-like characteristics, including expression of stem cell markers, multilineage differentiation and capacity to initiate orthotopic tumors in immunocompromised mice (NSG) at low cell numbers. The PBT030-2 EGFP:ffLuc TS-initiated xenograft model (0.1x10⁶ cells; 5 day engraftment) has been previously used to evaluate in vivo anti-tumor activity in NSG mice of IL13Rα2-specificCAR expressing T cells, whereby three injections of 2x10⁶ cytolytic T lymphocytes (CTLs) over a course of 2 weeks were shown to reduce tumor growth. However, in those experiments the majority of the PBT030-2 tumors eventually recurred. By comparison, a single injection of IL13(EQ)BBγ/CD19t+ T_{CM} (1.1x10⁶ CAR+ T_{CM}; 2x10⁶ total TCM) exhibited robust anti-tumor activity against PBT030-2 EGFP:ffLuc TS-initiated tumors (0.1x10⁶ cells; 5 day engraftment) as shown in **Figures 8A-C.** As compared to NSG mice treated with either PBS or mock transduced T_{CM} (no CAR), IL13(EQ)BBγ/CD19t+ T_{CM} significantly reduce ffLuc flux (p < 0.001 at >18-days) and significantly improve survival (p = 0.0008).

Briefly, EGFP-ffLuc+ PBT030-2 tumor cells (1×10⁵) were stereotactically implanted into the right forebrain of NSG mice. On day 5, mice received either 2x10⁶ IL13(EQ)BBγ/CD19t+ T_{CM} (1.1x106 CAR+; n=6), 2x10⁶ mock T_{CM} (no CAR; n=6) or PBS (n=6). **Figure 8A** depicts representative mice from each group showing relative tumor burden using Xenogen Living Image. Quantification of ffLuc flux (photons/sec) shows that IL13(EQ)BBζ/CD19t+ T_{CM} induce tumor regression as compared tomock-transduced T_{CM} and PBS (#p<0.02, *p<0.001, repeated measures ANOVA) **(****Figure 8B****).** As shown in **Figure 8C****,** a Kaplan Meier survival curve (n=6 per group) demonstrates significantly improved survival (p=0.0008; log-rank test) for mice treated with IL13(EQ)BBγ/CD19t+ T_{CM}.

### Example 8: Comparison of IL13(EQ)BBζ+ Tcm and Non-Tcm IL13-zetakine CD8+ CTL Clones in Antitumor Efficacy and T cell Persistence

The studies described below compare IL13(EQ)BBζ+ Tcm and a previously created IL13Rα2-specific human CD8+ CTLs (IL13-zetakine CD8+ CTL (described in Brown et al. 2012 Clin Cancer Res 18:2199 and Kahlon et al. 2004 Cancer Res 64:9160). The IL13-zetakine uses a CD3ζ stimulatory domain, lacks a co-stimulatory domain and uses the same IL13 variant as IL13(EQ)BBζ+.

A panel of primary lines (PBT) from patient glioblastoma specimens grown as tumor spheres (TSs) in serum-free media was generated (Brown et al. 2012 Clin Cancer Res 18:2199; Brown et al. 2009 Cancer Res 69:8886). These expanded TS lines exhibit stem cell-like characteristics, including expression of stem cell markers, multi-lineage differentiation and capacity to initiate orthotopic tumors in immunocompromised mice (NSG) at low cell numbers. The IL13Rα2+ primary low-passage glioblastoma TS line PBT030-2, which has been engineered to express both EGFP and firefly luciferase (ffLuc) reporter genes (PBT030-2 EGFP:ffLuc) (Brown et al. 2012 Clin Cancer Res 18:2199) was used for the experiments outlined below.

First, a single dose (1x10⁶ CAR T cells) of IL13(EQ)BBζ+ Tcm product was compared to IL13-zetakine CD8+ CTL clones evaluated against day 8 PBT030-2 EGFP:ffuc TS-initiated xenografts (0.1x10⁶ cells). While both IL13Rα2-specific CAR T cells (IL13-zetakine CTL and IL13(EQ)BBζ Tcm) demonstrated antitumor activity against established PBT030-2 tumors as compared to untreated and mock Tcm (CAR-negative) controls **(****Figures 9A** and **9B****),** IL13(EQ)BBZ+ Tcm mediated significantly improved survival and durable tumor remission with mice living >150 days as compared to our first-generation IL13-zetakine CD8+ CTL clones **(****Figure 9C****).**

To further compare the therapeutic effectiveness of these two IL13Rα2-CAR T cell products, a dose titration of 1.0, 0.3 and 0.1x10⁶ CAR T cells against day 8 PBT030-2 EGFP:ffuc TS-initiated tumors was performed **(****Figures 10A-C).** The highest dose (1x10⁶) of IL13-zetakine CD8+ CTL cl. 2D7 mediated antitumor responses as measured by Xenogen flux in 3 of 6 animals **(****Figure 10C****),** but no significant antitumor responses were observed at lower CAR T cell doses. By comparison, injection of II,13(EQ)BBζ+ Tcm product mediated complete tumor regression in the majority of mice at all dose levels, including treatment with as few as 0.1x10⁶ CAR T cells. These data demonstrate that IL13(EQ)BBζ+ Tcm is at least 10-fold more potent than IL13-zetakine CD8+ CTL clones in antitumor efficacy. The improved anti-tumor efficacy of is due to improved T cell persistence in the tumor microenvironment. Evaluation of CD3+ T cells 7-days post i.c. injection revealed significant numbers of IL13(EQ)BBζ+ Tcm in the tumor microenvironment, whereas very few first-generation IL13-zeta CTLs were present **(****Figure 11****).**

### Example 9: Comparison of CAR T cell delivery route for treatment of large TS-initiated PBT tumors

Described below are studies that compare the route of delivery, intraveneous (i.v.) or intracranial (i.c.), on antitumor activity against invasive primary PBT lines. In pilot studies (data not shown), it was unexpectedly observed that i.v. administered IL13(EQ)BBζ+ Tcm provided no therapeutic benefit as compared to PBS for the treatment of small (day 5) PBT030-2 EGFP:ffLuc tumors. This is in contrast to the robust therapeutic efficacy observed with i.c. administered CAR+ T cells. Reasoning that day 5 PBT030-2 tumors may have been too small to recruit therapeutic T cells from the periphery, a comparison was made of i.v. versus i.c. delivery against larger day 19 PBT030-2 EGFP:ffLuc tumors. For these studies, PBT030-2 engrafted mice were treated with either two i.v. infusions (5 x 10⁶ CAR+ Tcm; days 19 and 26) or four i.c. infusions (1 x 10⁶ CAR+ Tcm; days 19, 22, 26 and 29) of IL13(EQ)BBZ+ Tcm, or mock Tcm (no CAR). Here too no therapeutic benefit as monitored by Xenogen imaging or Kaplan-Meier survival analysis for i.v. administered CAR+ T cells **(****Figures 12A** and **12B****).** In contrast, potent antitumor activity was observed for i.c. administered IL13(EQ)BBζ+ Tcm **(****Figures 12A-B).** Next, brains from a cohort of mice 7 days post T cell injection were harvested and evaluated for CD3+ human T cells by IHC. Surprisingly, for mice treated i.v. with either mock Tcm or IL13(EQ)BBζ Tcm there were no detectable CD3+ human T cells in the tumor or in others mouse brain regions where human T cells typically reside (i.e. the leptomeninges) **(****Figure 12C****),** suggesting a deficit in tumor tropism. This is in contrast to the significant number of T cells detected in the i.c. treated mice **(Figure 12D).**

Tumor derived cytokines, particularly MCP-1/CCL2, are important in recruiting T cells to the tumor. Thus, PBT030-2 tumor cells were evaluated and it was found that this line produces high levels of MCP-1/CCL2 comparable to U251T cells (data not shown), a glioma line previously shown to attract i.v. administered effector CD8+ T cells to i.c. engrafted tumors. Malignant gliomas are highly invasive tumors and are often multifocal in presentation. The studies described above establish that IL13BBZ T_{CM} can eliminate infiltrated tumors such as PBT030-2, and mediate long-term durable antitumor activity. The capacity of intracranially delivered CAR T cells to traffic to multifocal disease was also examined. For this study PBT030-2 EGFP:ffLuc TSs were implanted in both the left and right hemispheres **(****Figure 13A****)** and CAR+ T cells were injected only at the right tumor site. Encouragingly, for all mice evaluated (n=3) we detected T cells by CD3 IHC 7-days post T cell infusion both at the site of injection (i.e. right tumor), as well within the tumor on the left hemisphere **(****Figure 13B****).** These findings provide evidence that CAR+ T cells are able to traffic to and infiltrate tumor foci at distant sites. Similar findings were also observed in a second tumor model using the U251T glioma cell line (data not shown).

### Example 10: IL13(EQ)BBζ/CD19t Sequences

The complete amino acid sequence of IL13(EQ)BBζ/CD19t is depicted in **Figure 17.** The entire sequence (SEQ ID NO:1) includes: a 22 amino acid GMCSF signal peptide (SEQ ID NO:2), a 112 amino acid IL-13 sequence (SEQ ID NO:3; amino acid substitution E13Y shown in bold); a 229 amino acid IgG4 sequence (SEQ ID NO:4; with amino acid substitutions L235E and N297Q shown in bold); a 22 amino acid CD4 transmembrane sequence (SEQ ID NO:5); a 42 amino acid 4-1BB sequence (SEQ ID NO:6); a 3 amino acid Gly linker; a 112 amino acid CD3ζ sequence (SEQ ID NO:7); a 24 amino acid T2A sequence (SEQ ID NO:8); and a 323 amino acid CD19t sequence (SEQ ID NO:9).

The mature chimeric antigen receptor sequence (SEQ ID NO:10) includes: a 112 amino acid IL-13 sequence (SEQ ID NO:3; amino acid substitution E13Y shown in bold); a 229 amino acid IgG4 sequence (SEQ ID NO:4; with amino acid substitutions L235E and N297Q shown in bold); at 22 amino acid CD4 sequence (SEQ ID NO:5); a 42 amino acid 4-1BB sequence (SEQ ID NO:6); a 3 amino acid Gly linker; and a 112 amino acid CD3ζ sequence (SEQ ID NO:7). Within this CAR sequence (SEQ ID NO:10) is the IL-13/IgG4/CD4t/41-BB sequence (SEQ ID NO:11), which includes: a 112 amino acid IL-13 sequence (SEQ ID NO:3; amino acid substitution E13Y shown in bold); a 229 amino acid IgG4 sequence (SEQ ID NO:4; with amino acid substitutions L235E and N297Q shown in bold); at 22 amino acid CD4 sequence (SEQ ID NO:5); and a 42 amino acid 4-1BB sequence (SEQ ID NO:6). The IL13/IgG4/CD4t/4-1BB sequence (SEQ ID NO:11) can be joined to the 112 amino acid CD3ζ sequence (SEQ ID NO:7) by a linker such as a Gly Gly Gly linker. The CAR sequence (SEQ ID NO:10) can be preceded by a 22 amino acid GMCSF signal peptide (SEQ ID NO:2). **Figure 18** depicts a comparison of the sequences of IL13(EQ)41BBζ[IL13{EQ}41BBζ T2A-CD19t_epHIV7; pF02630] (SEQ ID NO:12) and CD19Rop_epHIV7 (pJ01683) (SEQ ID NO:13).

## Claims

1. A population of human T cells transduced with a vector expressing a chimeric antigen receptor; wherein at least 50% of the transduced human T cells are central memory T cells;
wherein the central memory T cells are CD45RO+ and CD62L+ and comprise CD4+ and CD8+ cells;
wherein at least 10% of the transduced central memory T cells are CD4+ and at least 10% of the transduced central memory T cells are CD8+;
wherein the population of human T cells had been depleted of T cells that express CD25 and CD14 and cells that are CD45Ra+; and
wherein the depleted population of human T cells had been enriched for T cells expressing CD62L.

2. The population of human T cells of claim 1, wherein at least 15% of the central memory T cells are CD4+ and at least 15% are CD8+.

3. The population of human T cells of claim 1, wherein at least 50% of the transduced human T cells are CD4+ or CD8+.

4. The population of human T cells of claim 1, wherein at least 20% of the central memory T cells are CD4+.

5. The population of human T cells of claim 1, wherein at least 25% of the central memory T cells are CD4+.

6. The population of human T cells of claim 1, wherein at least 30% of the central memory T cells are CD4+.

7. The population of human T cells of claim 1, wherein at least 35% of the central memory T cells are CD4+.

8. A pharmaceutical composition comprising the population of human T cells of any of claims 1-7 for use in treating cancer in a patient in need thereof.

9. The pharmaceutical composition for use of claim 8, wherein the population of human T cells are autologous to the patient, or wherein the population of human T cells are allogenic to the patient.

## Patentansprüche

1. Population menschlicher T-Zellen, transduziert mit einem Vektor, der einen chimären Antigenrezeptor exprimiert; wobei mindestens 50 % der transduzierten menschlichen T-Zellen zentrale Gedächtnis-T-Zellen sind, wobei die zentralen Gedächtnis-T-Zellen CD45RO+ und CD62L+ sind und CD4+- und CD8+-Zellen umfassen,
wobei mindestens 10 % der transduzierten zentralen Gedächtnis-T-Zellen CD4+ sind und mindestens 10 % der transduzierten zentralen Gedächtnis-T-Zellen CD8+ sind,
wobei die Population menschlicher T-Zellen an T-Zellen, die CD25 und CD 14 exprimieren, und Zellen, die CD45Ra+ sind, abgereichert sind und
wobei die abgereicherte Population menschlicher T-Zellen hinsichtlich CD62L exprimierender T-Zellen angereichert ist.

2. Population menschlicher T-Zellen nach Anspruch 1, wobei mindestens 15 % der zentrale Gedächtnis-T-Zellen CD4+ sind und mindestens 15 % CD8+ sind.

3. Population menschlicher T-Zellen nach Anspruch 1, wobei mindestens 50 % der transduzierten menschlichen T-Zellen CD4+ oder CD8+ sind.

4. Population menschlicher T-Zellen nach Anspruch 1, wobei mindestens 20 % der zentralen Gedächtnis-T-Zellen CD4+ sind.

5. Population menschlicher T-Zellen nach Anspruch 1, wobei mindestens 25 % der zentralen Gedächtnis-T-Zellen CD4+ sind.

6. Population menschlicher T-Zellen nach Anspruch 1, wobei mindestens 30 % der zentralen Gedächtnis-T-Zellen CD4+ sind.

7. Population menschlicher T-Zellen nach Anspruch 1, wobei mindestens 35 % der zentralen Gedächtnis-T-Zellen CD4+ sind.

8. Pharmazeutische Zusammensetzung, umfassend die Population menschlicher T-Zellen nach einem der Ansprüche 1-7 zur Verwendung bei der Behandlung von Krebs bei einem dessen bedürftigen Patienten.

9. Pharmazeutische Zusammensetzung zur Verwendung nach Anspruch 8, wobei die Population menschlicher T-Zellen autolog zum Patienten ist oder wobei die Population menschlicher T-Zellen allogen zum Patienten ist.

## Revendications

1. Population de cellules T humaines transduites avec un vecteur exprimant un récepteur antigénique chimérique ; dans laquelle au moins 50 % des cellules T humaines transduites sont des cellules T à mémoire centrale ;
dans laquelle les cellules T à mémoire centrale sont CD45RO+ et CD62L+ et comprennent des cellules CD4+ et CD8+ ;
dans laquelle au moins 10 % des cellules T à mémoire centrale transduites sont CD4+ et au moins 10 % des cellules T à mémoire centrale transduites sont CD8+ ;
dans laquelle la population de cellules T humaines avait été appauvrie en cellules T qui expriment CD25 et CD14 et en cellules qui sont CD45Ra+ ; et
dans laquelle la population appauvrie de cellules T humaines avait été enrichie en cellules T exprimant CD62L.

2. Population de cellules T humaines selon la revendication 1, dans laquelle au moins 15 % des cellules T à mémoire centrale sont CD4+ et au moins 15 % sont CD8+.

3. Population de cellules T humaines selon la revendication 1, dans laquelle au moins 50 % des cellules T humaines transduites sont CD4+ ou CD8+.

4. Population de cellules T humaines selon la revendication 1, dans laquelle au moins 20 % des cellules T à mémoire centrale sont CD4+.

5. Population de cellules T humaines selon la revendication 1, dans laquelle au moins 25 % des cellules T à mémoire centrale sont CD4+.

6. Population de cellules T humaines selon la revendication 1, dans laquelle au moins 30 % des cellules T à mémoire centrale sont CD4+.

7. Population de cellules T humaines selon la revendication 1, dans laquelle au moins 35 % des cellules T à mémoire centrale sont CD4+.

8. Composition pharmaceutique comprenant la population de cellules T humaines selon l'une quelconque des revendications 1 à 7 pour une utilisation dans le traitement d'un cancer chez un patient qui en a besoin.

9. Composition pharmaceutique pour une utilisation selon la revendication 8, dans laquelle la population de cellules T humaines est autologue au patient, ou dans laquelle la population de cellules T humaines est allogénique au patient.
